# EUROPEAN PATENT APPLICATION

(11) **EP 4 628 576 A1**
(43) Date of publication of application: **08.10.2025**
(21) Application number: 24168875.3
(22) Date of filing: 07.04.2024
(51) Int. Cl.: C12N 7/00, C12N 15/11

(54) **MICRORNA MOLECULES FOR USE IN METHODS FOR IMPROVING RECOMBINANT AAV PRODUCTION**

(71) Applicant: Ascend Advanced Therapies GmbH, 82152 München (DE)
(72) Inventor: Hörer, Markus, München (DE); Schulze, Andreas, München (DE); Sonntag, Florian, München (DE); Finkbeiner, Bettina, Muenchen (DE); Otte, Kerstin, Biberach (DE); Burkhart, Madina, Biberach (DE)
(74) Representative: Kosti, Vasiliki

(57) **Abstract**

The present invention relates to microRNA (miRNA molecules) for use in methods of improving and/or increasing the yield of recombinant adeno-associated virus (AAV) production and uses of such miRNA molecules. The invention also relates to a method of identifying miRNA molecules capable to improve and/or increase the yield of recombinant adeno-associated virus (AAV) production.

## Description

### FIELD OF THE DISCLOSURE

The present invention relates to microRNA (miRNA) molecules for use in methods of improving and/or increasing the yield of recombinant adeno-associated virus (rAAV) production and uses of such miRNA molecules. The invention also relates to a method of identifying miRNA molecules capable to improve and/or increase the yield of recombinant adeno-associated virus rAAV production.

### BACKGROUND

Adeno-associated virus (AAV) is a member of the Parvoviridae family. The AAV genome is composed of a linear single-stranded DNA molecule which contains approximately 4.7 kilobases (kb) and consists of two major open reading frames encoding the non-structural Rep (replication) and structural Cap (capsid) proteins. Flanking the AAV coding regions are two cis-acting inverted terminal repeat (ITR) sequences, approximately 145 nucleotides in length, with interrupted palindromic sequences that can fold into hairpin structures that function as primers during initiation of DNA replication. In addition to their role in DNA replication, the ITR sequences have been shown to be necessary for viral integration, rescue from the host genome, and encapsidation of viral nucleic acid into mature virions (Muzyczka; 1992; Curr. Top. Micro. Immunol.; 158:97-129).

Multiple serotypes of AAV exist and offer varied tissue tropism. Known serotypes include, for example, AAV1, AAV2, AAV3, AAV4, AAV5, AAV6, AAV7, AAV8, AAV9, AAV 10 and AAV11.

Recombinant adeno-associated virus (rAAV) vectors are a leading gene delivery platform, have remarkable potential for gene therapy due to their promising safety profile and their ability to transduce many tissues *in vivo,* and several rAAV-mediated therapies have recently been approved (Ameri; 2018; J. Curr. Ophthalmol. 30, 1-2; Ylä-Herttuala; 2012; Mol. Ther. 20:1831-1832). However, despite these advances in the clinic, rAAV vector manufacturing remains a challenge. As an example, a phase 1/2 trial for hemophilia B required over 400 ten-layer cell stacks to generate sufficient material for six patients (Allay et al; 2011; Hum. Gene Ther. 22: 595-604). Although the trial was successful, this study highlights the need for new methods to improve vector generation. Increased production efficiency will reduce manufacturing costs, improve patient access, and make this emerging modality more feasible for large disease indications. Higher gene therapy vector loads are crucial to account for larger patient cohorts systemic diseases, or diseases in less accessible body sites. Thus, beyond the efforts in improving safety, alternative host cells, and alternative viral helpers, an additional area of rAAV research has been in scale-up, moving production from laboratory scale to industrial scale (Clement; 2016; Mol Ther Methods Clin Dev; 3:16002). An important focus of the rAAV vector development field has been to fine-tune the manufacturing process to augment the vector yield, purity, or its potency so that dose of vectors required per patient is low. rAAVtransduction requires a reasonable multiplicity of infection of ~10³ to 10⁵ vector genomes (vg) per cell depending on cell type. However, for a clinical trial, an estimated 10¹² to 10¹⁴ viral particles are essential to be efficacious during gene transfer. This high vector dose requirement in the clinical settings has underscored the need for optimizing vector production.

Taken together there remains a need to develop methods to improve and/or increase yield in the manufacture of an rAAV pharmaceutical product, while retaining quality standards e.g. low levels of empty capsids, host cell protein, and/or contaminating DNA.

### SUMMARY OF THE INVENTION

The present invention relates to microRNA (miRNA) molecules for use in methods of improving and/or increasing the yield of recombinant adeno-associated virus (rAAV) production and uses of such miRNA molecules. The invention also relates to a method of identifying miRNA molecules capable to improve and/or increase the yield of rAAV production.

Applicant has designed a method of identifying microRNAs which improve and/or increase the yield of AAV production. Such microRNAs can be used in methods where improved and/or increased AAV production is needed e.g. in gene therapy where AAV production plasmids systems are used as tools for transferring and expressing desired genetic material, such as the split plasmid system disclosed in WO2020/208379 A1, EPEP3722434 B1 and WO 2022/079429 A1 (incorporated herein by reference).

Based on the disclosure provided herein, those skilled in the art will recognize, or be able to ascertain using no more than routine experimentation, many equivalents to the specific embodiments of the invention described herein. Such equivalents are intended to be encompassed by the following embodiments (E).

Specifically, the present disclosure provides the following aspects, advantageous features and specific embodiments, respectively alone or in combination:
E1. A method for improving and/or increasing the viral genome titre, capsid titre and/or potency of recombinant adeno-associated virus (rAAV) produced during recombinant AAV production comprising the steps of
   - transfecting a host cell line with a synthetic microRNA molecule or a microRNA expressing vector
   - transfecting said cell line with a recombinant AAV production plasmid system, preferably a two - plasmid system
   - culturing said host cell line under conditions suitable for recombinant AAV production
   - obtaining recombinant AAV particles from said transfected cells,
   wherein said increase is at least 1.05 fold, at least, 1.1 fold, at least 2 fold, at least 3 fold, at least 4 fold, at least 5 fold or at least 6 fold compared to a corresponding method using without transfection with said same synthetic microRNA molecule or said same microRNA expressing plasmid.
E2. The method of E1, wherein said miRNA expressing plasmid comprises at least 2, at least 3, or at least 4 copies of a sequence encoding said miRNA.
E3. The method of E1 or E2 wherein said miRNA has a sequence with at least 80% sequence identity with any one of SEQ ID NOs: 1 to 4.
E4. The method of any one of E1 to E3 wherein said host cell line is a HEK293 cell line, preferably a suspension HEK293 cell line.
E5. The method of any one of E1 to E4 wherein said two- plasmid system comprises a helper plasmid comprising at least one adeno-associated virus (AAV) rep gene encoding at least one functional AAV Rep protein and at least one helper virus gene, and which does not comprise a cap gene encoding a functional set of Cap proteins, and a vector plasmid comprising an AAV cap gene encoding at least one functional AAV Cap protein.
E6. The method of any one of E1 to E5 wherein said AAV is a AAV S3 serotype.
E7. A microRNA molecule for use in a method of increasing the viral genome titre, capsid titre and/or potency of recombinant adeno-associated virus (rAAV) produced during recombinant AAV production according to anyone of E1 to E6, wherein said microRNA has a sequence with at least 80% identity with anyone of SEQ ID NOs: 1 to 4.
E8. A cell line according to anyone of claims 1 to 5 stably expressing a microRNA according to E7.
E9. Use of a miRNA molecule in a method for increasing the viral genome titre, capsid titre and/or potency of recombinant adeno-associated virus (rAAV) produced during recombinant AAV production, wherein said increase is at least 1.05 fold, at least 1.1 fold, at least 2 fold, at least 3 fold, at least 4 fold, at least 5 fold or at least 6 fold compared to a corresponding method without transfection with said same microRNA, preferably said microRNA having a sequence with at least 80 % identity with any one of SEQ ID NOs: 1 to 4.
E10. A method of identifying a microRNA which increases the viral genome titre, capsid titre and/or potency of recombinant adeno-associated virus (rAAV) produced during recombinant AAV production comprising the steps of
   - transfecting a first host cell line with a synthetic microRNA molecule or a microRNA expressing vector
   - transfecting said host cell line with a recombinant AAV plasmid system, preferably a two -plasmid system
   - culturing said cell line under conditions suitable for recombinant AAV production
   - obtaining recombinant AAV particles from said transfected cells,
   - analysing the functionality of said AAV particles, preferably by transducing a second cell line with a lysate obtained after lysis of said AAV particles, wherein said second cell line is selected based on the AVV serotype specificity, and
   wherein said increase of said is at least 1.05 fold, at least 1,1 fold, at least 2 fold, at least 3 fold, at least 4 fold, at least 5 fold or at least 6 fold compared to a corresponding method without transfection with said same synthetic microRNA molecule or said same microRNA expressing vector.
E11. The method of E10, wherein said microRNA expressing vector comprises at least 2, at least 3, or at least 4 copies of a sequence encoding said microRNA.
E12. The method of E10 or E11, wherein said miRNA has a sequence with at least 80% sequence identity with any one of SEQ ID NOs: 1 to 4.
E13. The method of any one of E10 to E12, wherein said first cell line is a HEK293 cell line, preferably a suspension HEK293 cell line.
E14. The method of any one of E10 to E13, wherein said two- plasmid system comprises a helper plasmid comprising at least one adeno-associated virus (AAV) rep gene encoding at least one functional AAV Rep protein and at least one helper virus gene, and which does not comprise a cap gene encoding a functional set of Cap proteins, and a vector plasmid comprising an AAV cap gene encoding at least one functional AAV Cap protein.
E15. The method of any one of E10 to E14, wherein said AAV is a AAV S3 serotype.
E16. The method of any one of E1 to E15, wherein said second cell line is a Huh7 cell line.

### BRIEF DESCRIPTON OF THE DRAWINGS

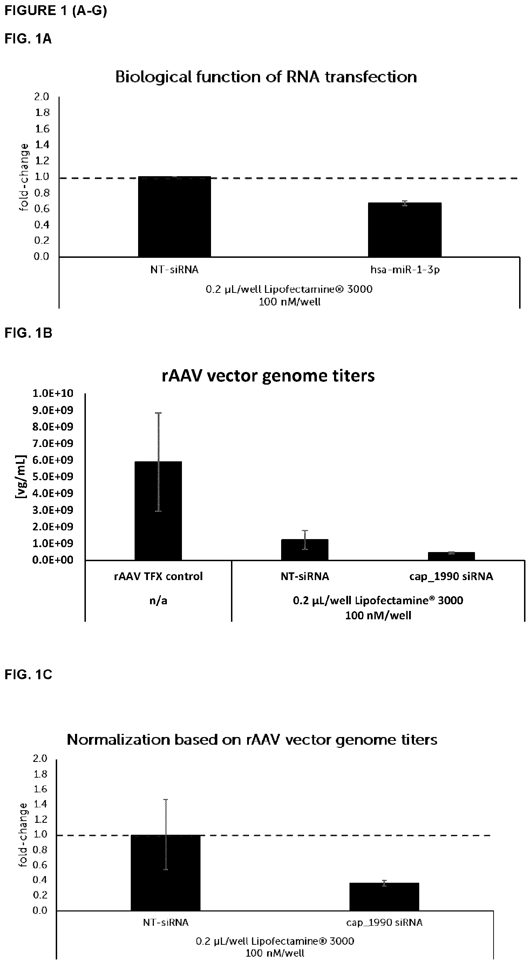

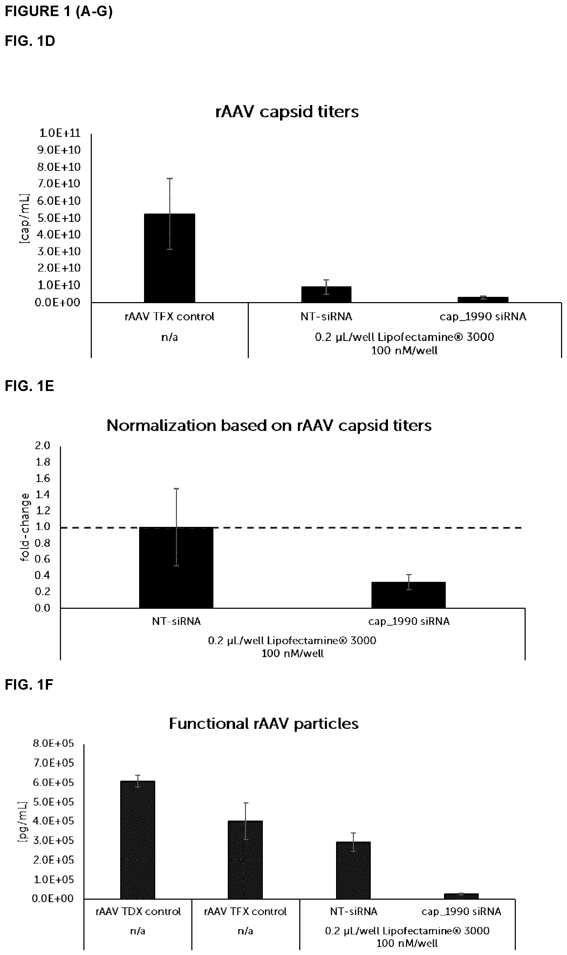

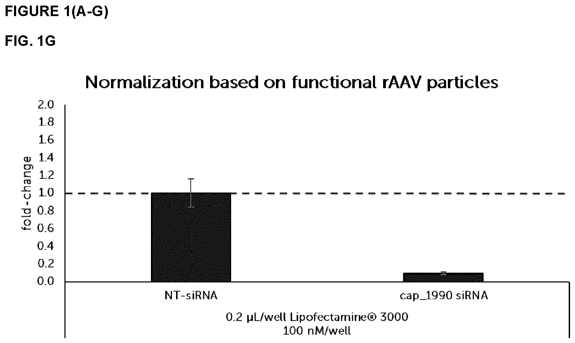

Figure 1 (A-G): miRNA screening process utilizing a two plasmid rAAV production system. Depicted are the biological function of transfected miRNA mimics (Fig. 1A), rAAV vector genome (Fig. 1B) and capsid titers (Fig. 1D) as well as functional rAAV particles (Fig. 1F). Additionally, the impact of cap_1990 siRNA as rAAV-specific trending control on rAAV vector genome (Fig. 1C) and capsid titers (Fig. 1E) as well as functional rAAV particles (Fig. 1G) is shown normalized against the respective NT-siRNA. Data are depicted as mean ±SD for two independent experiments for (Fig. 1A), (Fig. 1F) and (Fig. 1G). Data are depicted as mean ±SD (standard deviation) for biological triplicates for (Fig. 1B), (Fig. 1C), (Fig. 1D) and (Fig. 1E). The dashed line represents the NT-siRNA set to 1.

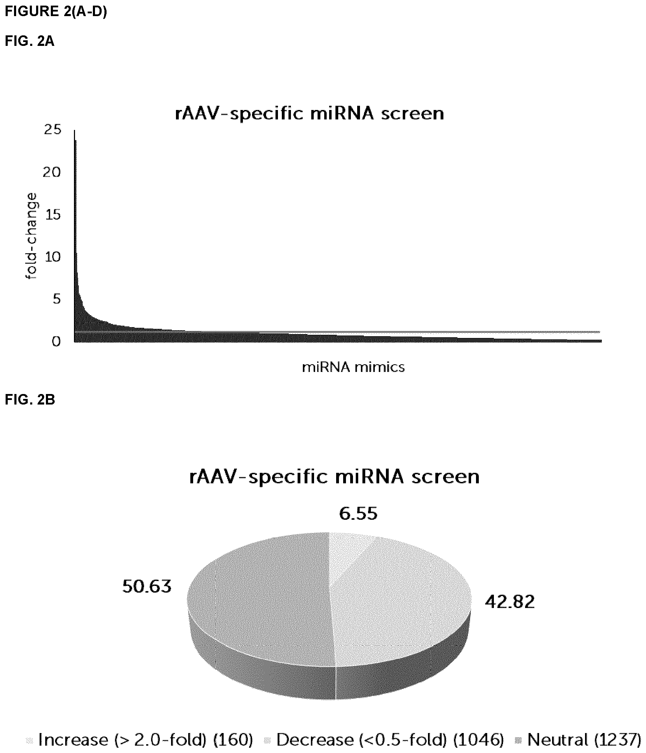

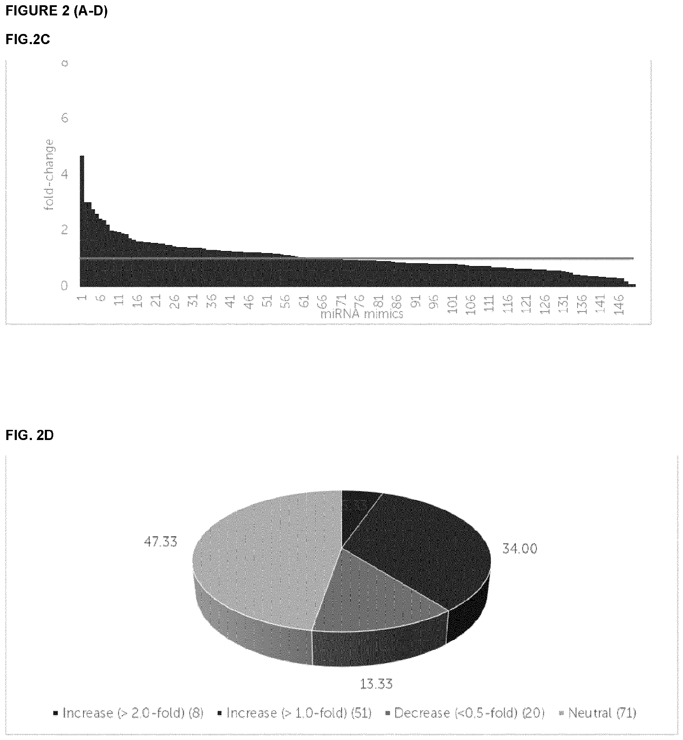

Figure 2 (A-D): Overview of miRNA-mediated effects on the production of functional rAAV particles in the rAAV-specific miRNA screen. (Fig. 2A) and (Fig. 2C) depict the normalization of miRNA-mediated effects on rAAV production in relation to the NT-siRNA. Data are depicted as mean of biological triplicates and were ordered according to the degree of quantitative change from strong increase to strong decrease. (Fig. 2B) and (Fig. 2D) depict percentages of miRNAs inducing positive, negative or no changes in rAAV production. Data for the complete rAAV-specific miRNA screen is shown in (Fig. 2A), (Fig. 2 B), whereas data for MP01 to MP06 is shown in (Fig. 2C) and (Fig. 2D).

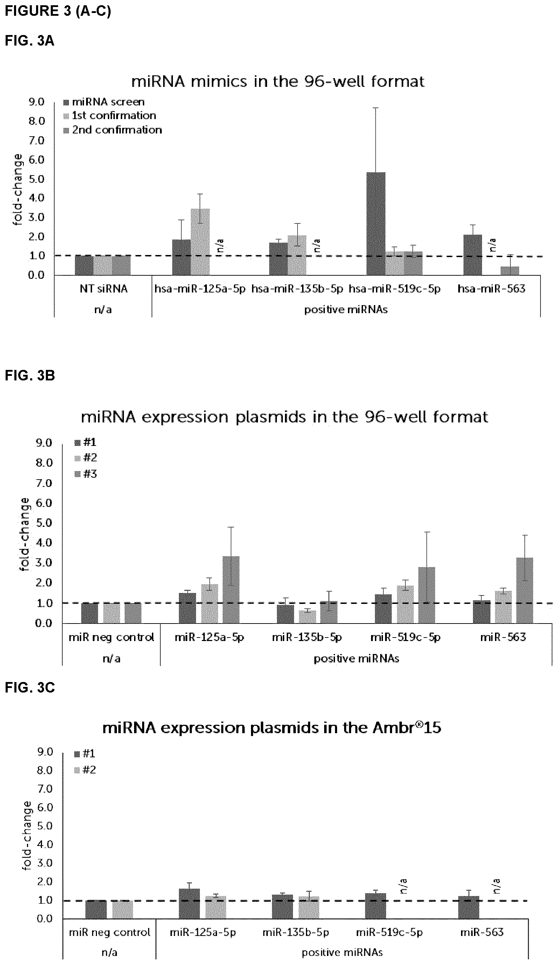

Figure 3 (A-C): Transient validation of selected miRNAs from the rAAV-specific miRNA screening in three transient validation systems. Fig. 3A. Validation of four selected miRNAs via transient miRNA mimic transfections in the 96-well format. Fig. 3B. Validation of selected miRNAs via transient miRNA expression plasmid transfections in the 96-well format. Fig. 3C. Validation of selected miRNAs via transient miRNA expression plasmid transfections in the Ambr@15. All miRNA- mediated effects on rAAV production are shown in relation to the miR-negative control expression plasmid (miR neg control). Data are depicted as mean ±SD for biological triplicates.

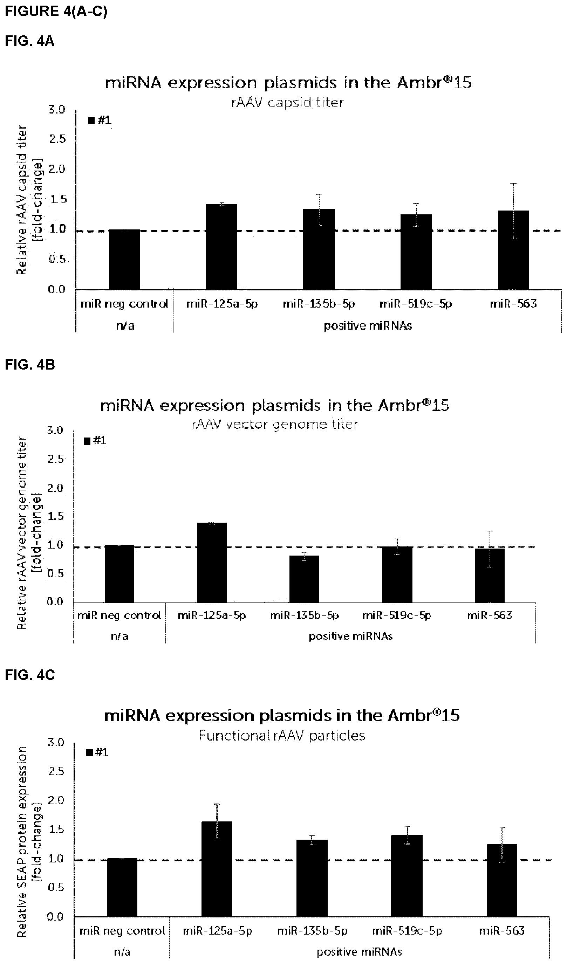

Figure 4 (A-C): Effect of selected miRNA expression plasmids in a two plasmid rAAV system on rAAV quantity in the Ambr@15 microbioreactor. (Fig. 4A) Effect of miRNA overexpression on the rAAV capsid titer. (Fig. 4B) Effect of miRNA overexpression on the rAAV vector genome titer. (Fig. 4C) Effect of miRNA overexpression on the produced functional rAAV particles. All miRNA-mediated effects on rAAV production are shown in relation to the miR-negative control expression plasmid (miR neg control).

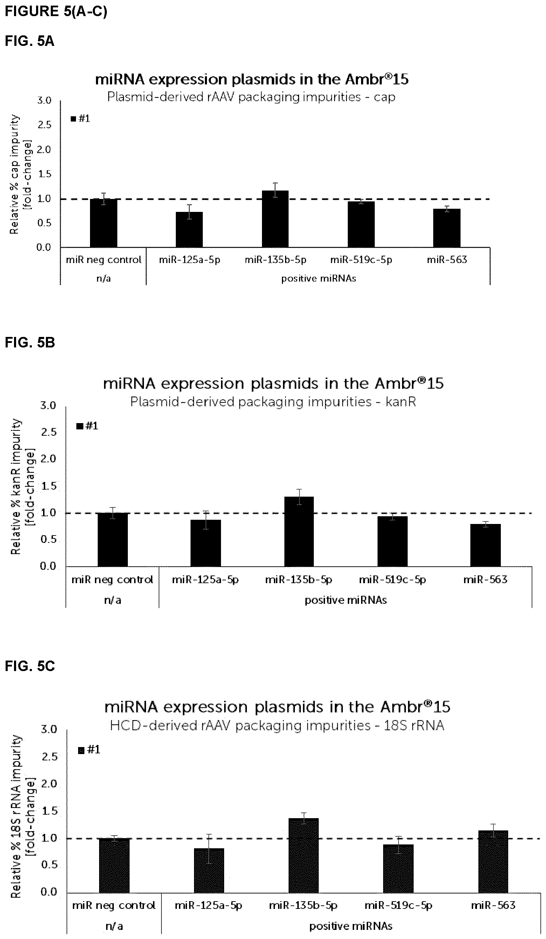

Figure 5 (A-C): Effect of selected miRNA expression plasmids in a two plasmid rAAV plasmid system on rAAV quality in the Ambr@15 microbioreactor. (Fig. 5A) Effect of miRNA overexpression on plasmid-derived cap rAAV packaging impurities. (Fig. 5B) Effect of miRNA overexpression on plasmid-derived kanR rAAV packaging impurities. (Fig. 5C) Effect of miRNA overexpression on host cell-derived 18S rRNA rAAV packaging impurities. All miRNA-mediated effects on rAAV production are shown in relation to the miR-negative control expression plasmid (miR neg control).

### DETAILED DESCRIPTION

The present invention relates to microRNA (miRNA molecules) for use in methods of improving and/or increasing the yield of recombinant adeno-associated virus (AAV) production and uses of such miRNA molecules. The invention also relates to a method of identifying miRNA molecules capable to increase the yield of recombinant adeno-associated virus (AAV) production.

MicroRNAs (miRNAs) are endogenous small non-coding RNA molecules of about 22 nucleotides, that post-transcriptionally regulate gene expression in eukaryotic cells and are highly conserved across species. A single miRNA usually regulates up to hundreds of different messenger RNAs (mRNAs) and most mRNAs are expected to be targeted by multiple miRNAs. miRNA genes are transcribed by RNA polymerase-II and subsequently processed, giving rise to single-stranded mature miRNAs, which are incorporated into the RNA-induced silencing complex (RISC). As a central part of the RISC complex, the miRNA guides RISC to its mRNA targets, where the miRNA binds the 3'-untranslated region (3'UTR) of the mRNA transcript by partial complementary base pairing. Gene silencing occurs either through argonaute-2 (AG02)-mediated mRNA cleavage or translational repression facilitated by AG01 to 4, with both ways finally reducing the levels of corresponding proteins (van Rooij, 2011). In contrast to small interfering RNAs (siRNAs), miRNAs are only partially complementary to binding sites within the 3'UTR of the target transcript, leading to less specificity and, thus, increasing the pool of potential target genes. Complete Watson-Crick base pairing is only compulsory/ at the miRNA "seed" region which covers the sequence between nucleotide 2 to 7/8 at the 5-end of the mature miRNA. miRNAs with identical "seed" sequences such as the miR-30 members are grouped into families. According to bioinformatic target prediction tools, members of the same family are supposed to share a large number of target mRNAs.

MicroRNAs are known to play a major role in viral infectivity and pathogenicity. Their effect on virus life cycle emanates from complex interactions involving host microRNAs or virally encoded microRNAs and their intracellular targets (Gottwein et al;2008; Cell Host Microbe.; 3(6):375-387). miR-122, a liver-specific microRNA with its target in the 5'-untranslated region (UTR) of hepatitis C virus (HCV) enhances the viral RNA translation and also protects HCV RNA genome from the exoribonucleases (Sedano et al; 2015; Semin Liver Dis.; 35(1):75-80). Alternatively, certain atypical eukaryotic DNA to microRNA interactions, such as the role of miR-373 inducing the gene expression from E-cadherin promoter shows the possibility that promoter associated microRNAs could lead to increased gene expression from viral genes (Place et al; 2008; Proc Natl Acad Sci USA; 105(5):1608-1613). However, there are no studies available on the role of small RNAs, suitably miRNAs, during recombinant AAV manufacturing.

The inventors of the instant disclosure have designed a high-throughput method of screening a miRNA library in order to identify miRNA molecules which can induce an improved and/or increased yield of *in vitro* rAAV production. Such miRNAs are useful in methods where improved and/or increased yield of rAAV production is essential such as in large scale gene therapy manufacturing where rAAV production systems are utilized.

In one aspect, the present disclosure relates to a method for producing a preparation comprising recombinant adeno-associated virus (rAAV) particles, wherein said method comprises a step of introducing a microRNA molecule or a microRNA expressing vector to a host cell under suitable conditions so that said microRNA molecule is overexpressed; and a step of culturing said host cell under suitable conditions to produce the preparation.

In one embodiment said method is a method for producing a preparation comprising rAAV particles having improved and/or increased viral genome titre, capsid titre and/or potency compared to a preparation using a corresponding method without introducing said same microRNA molecule or said same microRNA expressing vector. In one embodiment said increase is at least 1.05 fold, at least 1,1 fold, at least 2 fold, at least 3 fold, at least 4 fold, at least 5 fold or at least 6 fold compared to a preparation using a corresponding method without introducing said same microRNA molecule or said same microRNA expressing vector.

The introduction of a miRNA or a miRNA expressing vector to a host cell is possible by different means known to those skilled in the art, for example by transfection, i.e. non-viral methods for transferring a nucleic acid molecule into eukaryotic cells. Alternatively, a miRNA or miRNA expressing vector can be introduced into a cell by transduction, i.e. by a virus-mediated transfer of the nucleic acid. For such applications, the nucleic acid construct is preferably provided as a viral vector.

In specific cases, for the introduction of said miRNA, it may be preferred that said miRNA is expressed through integration of a copy or copies of said miRNA encoding sequence into the cell's genome. By introducing said copy or copies of said miRNA encoding sequence according to the invention into the cell's endogenous genome, a stable cell line for biopharmaceutical manufacturing can be provided. Such cell lines produce biomolecules at constant and reliable amounts and are, thus, particularly preferred for large scale productions, which are usually operated to provide established and highly demanded biopharmaceuticals. Moreover, by integrating said copy or copies of said miRNA encoding sequence into the genome, the probability of losing gene expression during continuous cell proliferation is reduced and said sequence is present throughout the entire culture's lifetime. Accordingly, the cell is preferably a stable cell line cell.

In a preferred embodiment, said miRNA expressing vector is introduced into the cell by transfection. Transient transfection is an easy and fast way to provide a given cell with new properties. It is neither labour nor cost intensive and does not need extensive selection processes. Introducing said miRNA expressing vector by transfection is particularly preferred where biomolecules need to be produced on short term notice or when small amounts of the biomolecule are needed, such that the labour-intensive establishment of a stable cell line would be inefficient.

Thus, in one embodiment the present disclosure relates to a method for producing a preparation comprising recombinant adeno-associated virus (rAAV) particles, wherein said method comprises the steps of
- transfecting a host cell line, preferably a mammalian cell line, more preferably a human cell line, with a synthetic microRNA molecule or a microRNA expressing vector
- transfecting said host cell line with a recombinant AAV production plasmid system, preferably a three plasmid AAV production system or a two plasmid AAV production system
- culturing said cell line under conditions suitable for recombinant AAV production
- obtaining recombinant AAV particles from said transfected cells.

In one embodiment said method is a method for producing a preparation comprising rAAV having improved and/or increased viral genome titre, capsid titre and/or potency compared to an rAAV using a corresponding method without transfection with said same synthetic microRNA molecule or said same microRNA expressing vector.

In one embodiment said increase is at least 1.05 fold, at least 1,1 fold, at least 2 fold, at least 3 fold, at least 4 fold, at least 5 fold or at least 6 fold compared to a corresponding method without transfection with said same synthetic microRNA molecule or said same microRNA expressing vector. In one embodiment said recombinant AAV production plasmid system is a three plasmid AAV production system.

In one embodiment said recombinant AAV production plasmid system is a two plasmid AAV production system.

In one embodiment said miRNA expressing vector comprises at least 2, at least 3, or at least 4 copies of a sequence encoding said microRNA.

In one embodiment said microRNA has a sequence with at least 80%, or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with any one of SEQ ID NOs: 1 to 4. In one embodiment said microRNA comprises or consists of any one of SEQ ID NOs: 1 to 4.

In one embodiment said host cell line is a HEK293 cell line, preferably a suspension HEK293 cell line.

In one embodiment said two- plasmid system comprises a helper plasmid comprising at least one adeno-associated virus (AAV) rep gene encoding at least one functional AAV Rep protein and at least one helper virus gene, and which does not comprise a cap gene encoding a functional set of Cap proteins, and a vector plasmid comprising an AAV cap gene encoding at least one functional AAV Cap protein.

In one embodiment said AAV is a AAV S3 serotype.

In one aspect the present invention relates to a method for improving and/or increasing the viral genome titre, capsid titre and/or potency of recombinant adeno-associated virus (rAAV) produced during recombinant AAV production comprising the steps of
- transfecting a host cell line, preferably a mammalian cell line, more preferably a human cell line, with a synthetic microRNA molecule or a microRNA expressing vector
- transfecting said host cell line with a recombinant AAV production plasmid system, preferably a three plasmid AAV production system or a two plasmid AAV production system
- culturing said cell line under conditions suitable for recombinant AAV production
- obtaining recombinant AAV particles from said transfected cells,
wherein said increase is at least 1.05 fold, at least 1.1 fold, at least 2 fold, at least 3 fold, at least 4 fold, at least 5 fold or at least 6 fold compared to a corresponding method without transfection with said same synthetic microRNA molecule or a said same microRNA expressing vector.

In one embodiment, said method for improving and/or increasing the viral genome titre, capsid titre and/or potency of recombinant adeno-associated virus (rAAV) produced during recombinant AAV production comprises the steps of
- transfecting a mammalian cell line, preferably a human cell line, with a synthetic microRNA molecule or a microRNA expressing vector
- transfecting said cell line with a three plasmid AAV production system
- culturing said cell line under conditions suitable for recombinant AAV production
- obtaining recombinant AAV particles from said transfected cells,
wherein said increase is at least 1.05 fold, at least 1 fold, at least 2 fold, at least 3 fold, at least 4 fold, at least 5 fold or at least 6 fold compared to a corresponding method without transfection with said same synthetic microRNA molecule or said same microRNA expressing vector.

In one embodiment said method for improving and/or increasing the viral genome titre, capsid titre and/or potency of recombinant adeno-associated virus (rAAV) produced during recombinant AAV production comprises the steps of
- transfecting a mammalian cell line, preferably a human cell line, with a synthetic microRNA molecule or a microRNA expressing vector
- transfecting said cell line with a two plasmid AAV production plasmid system
- culturing said cell line under conditions suitable for recombinant AAV production
- obtaining recombinant AAV particles from said transfected cells,
wherein said increase is at least 1.05 fold, at least 1,1 fold, at least 2 fold, at least 3 fold, at least 4 fold, at least 5 fold or at least 6 fold compared to a corresponding method without transfection with said same synthetic microRNA molecule or said same microRNA expressing vector.

In one embodiment said method for improving and/or increasing the viral genome titre, capsid titre and/or potency of recombinant adeno-associated virus (rAAV) produced during recombinant AAV production comprises the steps of
- transfecting a mammalian cell line, preferably a human cell line, with a synthetic microRNA molecule
- transfecting said cell line with a recombinant AAV production plasmid system, preferably a three plasmid AAV production system or a two plasmid AAV production system
- culturing said cell line under conditions suitable for recombinant AAV production
- obtaining recombinant AAV particles from said transfected cells,
wherein said increase is at least 1.05 fold, at least 1.1 fold, at least 2 fold, at least 3 fold, at least 4 fold, at least 5 fold or at least 6 fold compared to a corresponding method without transfection with said same synthetic microRNA molecule.

In one embodiment said method for improving and/or increasing the viral genome titre, capsid titre and/or potency of recombinant adeno-associated virus (rAAV) produced during recombinant AAV production comprises the steps of
- transfecting a mammalian cell line, preferably a human cell line, with a synthetic microRNA molecule
- transfecting said cell line with a three plasmid AAV production system
- culturing said cell line under conditions suitable for recombinant AAV production
- obtaining recombinant AAV particles from said transfected cells,
wherein said increase is at least 1.05 fold, at least 1.1 fold, at least 2 fold, at least 3 fold, at least 4 fold, at least 5 fold or at least 6 fold compared to a corresponding method without transfection with said same synthetic microRNA molecule.

In one embodiment said method for improving and/or increasing the viral genome titre, capsid titre and/or potency of recombinant adeno-associated virus (rAAV) produced during recombinant AAV production comprises the steps of
- transfecting a mammalian cell line, preferably a human cell line, with a synthetic microRNA molecule
- transfecting said cell line with a two -plasmid AAV production system
- culturing said cell line under conditions suitable for recombinant AAV production
- obtaining recombinant AAV particles from said transfected cells,
wherein said increase is at least 1.05 fold, at least 1.1 fold, at least 2 fold, at least 3 fold, at least 4 fold, at least 5 fold or at least 6 fold compared to a corresponding method without transfection said same synthetic microRNA molecule.

In one embodiment said method for improving and/or increasing the viral genome titre, capsid titre and/or potency of recombinant adeno-associated virus (rAAV) produced during recombinant AAV production comprises the steps of
- transfecting a mammalian cell line, preferably a human cell line, with a microRNA expressing vector
- transfecting said cell line with a recombinant AAV production plasmid system, preferably a three plasmid AAV production system or a two -plasmid AAV production system
- culturing said cell line under conditions suitable for recombinant AAV production
- obtaining recombinant AAV particles from said transfected cells,
wherein increase is at least 1.05 fold, at least 1 fold, at least 2 fold, at least 3 fold, at least 4 fold, at least 5 fold or at least 6 fold compared to a corresponding method without transfection with same said microRNA expressing vector.

In one embodiment said method for improving and /or increasing the viral genome titre, capsid titre and/or potency of recombinant adeno-associated virus (rAAV) produced during recombinant AAV production comprises the steps of
- transfecting a mammalian cell line, preferably a human cell line, with a microRNA expressing vector
- transfecting said cell line with a three -plasmid AAV production system
- culturing said cell line under conditions suitable for recombinant AAV production
- obtaining recombinant AAV particles from said transfected cells,
wherein said increase is at least 1.05 fold, at least 1.1 fold, at least 2 fold, at least 3 fold, at least 4 fold, at least 5 fold or at least 6 fold compared to a corresponding method without transfection with same said microRNA expressing vector.

In one embodiment said method for improving and/ or increasing the viral genome titre, capsid titre and/or potency of recombinant adeno-associated virus (rAAV) produced during recombinant AAV production comprises the steps of
- transfecting a mammalian cell line, preferably a human cell line, with a microRNA expressing vector
- transfecting said cell line with a two -plasmid AAV production system
- culturing said cell line under conditions suitable for recombinant AAV production
- obtaining recombinant AAV particles from said transfected cells,
wherein said increase is at least 1.05 fold, at least 1.1 fold, at least 2 fold, at least 3 fold, at least 4 fold, at least 5 fold or at least 6 fold compared to a corresponding method without transfection with said same microRNA expressing vector.

In one embodiment, said miRNA expressing vector comprises at least 2, at least 3, or at least 4 copies of a sequence encoding said miRNA.

In one embodiment, said miRNA expressing vector comprises at least 2 copies of a sequence encoding said miRNA.

In one embodiment, said miRNA expressing vector comprises at least 3 copies of a sequence encoding said microRNA.

In one embodiment, said miRNA expressing vector comprises at least 4 copies of a sequence encoding said microRNA.

In one embodiment said microRNA has a sequence with at least 80%, or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with any one of SEQ ID NOs: 1 to 4. In one embodiment said microRNA comprises or consists of any one of SEQ ID NOs: 1 to 4.

In one embodiment said microRNA has a sequence with at least 80%, or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with SEQ ID NO: 1. In one embodiment said microRNA comprises or consists of SEQ ID NO: 1.

In one embodiment said microRNA has a sequence with at least 80%, or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with SEQ ID NO: 2. In one embodiment said microRNA comprises or consists of SEQ ID NO: 2.

In one embodiment said microRNA has a sequence with at least 80%, or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with SEQ ID NO: 3. In one embodiment said microRNA comprises or consists of SEQ ID NO: 3.

In one embodiment said microRNA has a sequence with at least 80%, or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with SEQ ID NO: 4. In one embodiment said microRNA comprises or consists of SEQ ID NO: 4.

In one embodiment, said host cell line is a HEK293 cell line, preferably a suspension HEK293 cell line.

In one embodiment said two plasmid system comprises a helper plasmid comprising at least one adeno-associated virus (AAV) rep gene encoding at least one functional AAV Rep protein and at least one helper virus gene, and which does not comprise a cap gene encoding a functional set of Cap proteins, and a vector plasmid comprising an AAV cap gene encoding at least one functional AAV Cap protein.

In one embodiment said AAV is a AAV S3 serotype.

In one aspect the invention relates to a microRNA molecule for use in a method for producing a preparation comprising recombinant adeno-associated virus (rAAV) particles or in a method of improving and/or increasing the viral genome titre, capsid titre and/or potency of recombinant adeno-associated virus (rAAV) produced during recombinant AAV production wherein said microRNA has a sequence with at least 80% or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% identity with anyone of SEQ ID NOs: 1 to 4. In one embodiment said microRNA comprises or consists of any one of SEQ ID NOs: 1 to 4.

In one embodiment said microRNA molecule for use in a method for producing a preparation comprising recombinant adeno-associated virus (rAAV) particles or in a method of improving and/or increasing the viral genome titre, capsid titre and/or potency of recombinant adeno-associated virus (rAAV) produced during recombinant AAV production, wherein said microRNA has a sequence with at least 80% or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% identity with SEQ ID NO: 1. In one embodiment said microRNA comprises or consists of any one of SEQ ID NOs: 1.

In one embodiment said microRNA molecule for use in a method for producing a preparation comprising recombinant adeno-associated virus (rAAV) particles or in a method of improving and/or increasing the viral genome titre, capsid titre and/or potency of recombinant adeno-associated virus (rAAV) produced during recombinant AAV production, wherein said microRNA has a sequence with at least 80% or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% identity with SEQ ID NO: 2. In one embodiment said microRNA comprises or consists of any one of SEQ ID NOs: 2.

In one embodiment said microRNA molecule for use in a method for producing a preparation comprising recombinant adeno-associated virus (rAAV) particles or in a method of improving and/or increasing the viral genome titre, capsid titre and/or potency of recombinant adeno-associated virus (rAAV) produced during recombinant AAV production, wherein said microRNA has a sequence with at least 80% or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% identity with SEQ ID NO: 3. In one embodiment said microRNA comprises or consists of any one of SEQ ID NOs: 3.

In one embodiment said microRNA molecule for use in a method for producing a preparation comprising recombinant adeno-associated virus (rAAV) particles or in a method of improving and/or increasing the viral genome titre, capsid titre and/or potency of recombinant adeno-associated virus (rAAV) produced during recombinant AAV production, wherein said microRNA has a sequence with at least 80% or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% identity with SEQ ID NO: 4. In one embodiment said microRNA comprises or consists of any one of SEQ ID NOs: 4.

In one aspect the invention relates to a host cell line for use in a method for producing a preparation comprising recombinant adeno-associated virus (rAAV) particles or in a method of improving and/or increasing the viral genome titre, capsid titre and/or potency of a recombinant adeno-associated virus (rAAV) produced during recombinant AAV production expressing a microRNA of the invention.

In one embodiment said host cell line for use in a method for producing a preparation comprising recombinant adeno-associated virus (rAAV) particles or in a method of improving and/or increasing the viral genome titre, capsid titre and/or potency of a recombinant adeno-associated virus (rAAV) produced during recombinant AAV production stably expresses a microRNA of the invention.

In one aspect the invention relates to a use of a microRNA molecule in a method for producing a preparation comprising recombinant adeno-associated virus (rAAV) particles or in a method for improving and/or increasing the viral genome titre, capsid titre and/or potency of recombinant adeno-associated virus (rAAV) produced during recombinant AAV production, wherein said increase is at least 1.05 fold, at least 1.1 fold, at least 2 fold, at least 3 fold, at least 4 fold, at least 5 fold or at least 6 fold compared to a corresponding method without using said same microRNA.

In one embodiment said increase is at least 1.05 fold, at least 1,1 fold, at least 2 fold, at least 3 fold, at least 4 fold, at least 5 fold or at least 6 fold compared to an equivalent method without using said microRNA, and said microRNA has a sequence with at least 80 % or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% identity with any one of SEQ ID NOs: 1 to 4. In one embodiment said increase is at least 1.05 fold, at least 1,1 fold, at least 2 fold, at least 3 fold, at least 4 fold, at least 5 fold or at least 6 fold compared to an equivalent method without using said same microRNA, and said microRNA comprises or consists of any one of SEQ ID NOs: 1 to 4.

In one embodiment said increase is at least 1.05 fold, at least 1.1 fold, at least 2 fold, at least 3 fold, at least 4 fold, at least 5 fold or at least 6 fold compared to an equivalent method without using said same microRNA, wherein said microRNA has a sequence with at least 80 % or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% identity with SEQ ID NO: 1. In one embodiment said increase of said is at least 1..05 fold, at least 1.1 fold, at least 2 fold, at least 3 fold, at least 4 fold, at least 5 fold or at least 6 fold compared to a corresponding method without using said same microRNA, and said microRNA comprises or consists of SEQ ID NO: 1.

In one embodiment said increase is at least 1.05 fold, at least 1.1 fold, at least 2 fold, at least 3 fold, at least 4 fold, at least 5 fold or at least 6 fold compared to a corresponding method without using said same microRNA, wherein said microRNA has a sequence with at least 80 % or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% identity with SEQ ID NO: 2. In one embodiment said increase is at least 1.05 fold, at least 1.1 fold, at least 2 fold, at least 3 fold, at least 4 fold, at least 5 fold or at least 6 fold compared to a corresponding method without using said same microRNA, and said microRNA comprises or consists of SEQ ID NO: 2.

In one embodiment said increase is at least 1.05 fold, at least 1.1 fold, at least 2 fold, at least 3 fold, at least 4 fold, at least 5 fold or at least 6 fold compared to a corresponding method without using said same microRNA, and said microRNA has a sequence with at least 80 % or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% identity with SEQ ID NO: 3. In one embodiment said increase is at least 1.05 fold, at least 1.1 fold, at least 2 fold, at least 3 fold, at least 4 fold, at least 5 fold or at least 6 fold compared to a corresponding method without using said same microRNA, and said microRNA comprises or consists of SEQ ID NO: 3.

In one embodiment said increase is at least 1.05 fold, at least 1.1 fold, at least 2 fold, at least 3 fold, at least 4 fold, at least 5 fold or at least 6 fold compared to a corresponding method without using said same microRNA, and said microRNA has a sequence with at least 80 % or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% identity with SEQ ID NO: 4. In one embodiment said increase is at least 1.05 fold at least 1.1 fold, at least 2 fold, at least 3 fold, at least 4 fold, at least 5 fold or at least 6 fold compared to a corresponding method without using said same microRNA, and said microRNA comprises or consists of SEQ ID NO: 4.

In another aspect the invention relates to a method of identifying a microRNA which improves and/or increases the viral genome titre, capsid titre and/or potency of recombinant adeno-associated virus (rAAV) produced during recombinant AAV production comprising the steps of
- transfecting a first host cell line with a synthetic microRNA molecule or a microRNA expressing vectror
- transfecting said cell line with a recombinant AAV plasmid system, preferably a three plasmid system or a two plasmid system
- culturing said cell line under conditions suitable for recombinant AAV production
- obtaining recombinant AAV particles from said transfected cells,
- analysing the production of functional rAAV particles preferably by transducing a second cell line with a lysate obtained after lysis of said first host cells, wherein said second cell line is selected based on the rAVV serotype specificity, and
wherein said increase of said is at least 1.05 fold, at least 1.1 fold, at least 2 fold, at least 3 fold, at least 4 fold, at least 5 fold or at least 6 fold compared to a corresponding method without transfection with said same synthetic microRNA molecule or said same microRNA expressing vector.

In one embodiment of said method of identifying a microRNA which improves and/or increases the viral genome titre, capsid titre and/or potency of recombinant adeno-associated virus (rAAV) produced during recombinant AAV production, said microRNA expressing plasmid comprises at least 2, at least 3, or at least 4 copies of a sequence encoding said microRNA.

In one embodiment of said method of identifying a microRNA which improves and/or increases the viral genome titre, capsid titre and/or potency of recombinant adeno-associated virus (rAAV) produced during recombinant AAV production, said microRNA has a sequence with at least 80% or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with any one of SEQ ID NOs: 1 to 4. In one embodiment of said method of identifying a microRNA which improves and/or increases the viral genome titre, capsid titre and/or potency of recombinant adeno-associated virus (rAAV) in a preparation produced during recombinant AAV production, said microRNA comprises or consists of any one of SEQ ID NOs: 1 to 4.

In one embodiment of said method of identifying a microRNA which improves and/or increases the viral genome titre, capsid titre and/or potency of recombinant adeno-associated virus (rAAV) in a preparation produced during recombinant AAV production, said microRNA has a sequence with at least 80% or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with SEQ ID NO: 1. In one embodiment of said method of identifying a microRNA which improves and/or increases the viral genome titre, capsid titre and/or potency of recombinant adeno-associated virus (rAAV) in a preparation produced during recombinant AAV production, said microRNA comprises or consists of SEQ ID NO: 1.

In one embodiment of said method of identifying a microRNA which improves and/or increases the viral genome titre, capsid titre and/or potency of recombinant adeno-associated virus (rAAV) in a preparation produced during recombinant AAV production, said microRNA has a sequence with at least 80% or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with SEQ ID NO: 2. In one embodiment of said method of identifying a microRNA which improves and/or increases the viral genome titre, capsid titre and/or potency of recombinant adeno-associated virus (rAAV) in a preparation produced during recombinant AAV production, said microRNA comprises or consists of SEQ ID NO: 2.

In one embodiment of said method of identifying a microRNA which improves and/or increases the viral genome titre, capsid titre and/or potency of recombinant adeno-associated virus (rAAV) in a preparation produced during recombinant AAV production, said microRNA has a sequence with at least 80% or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with SEQ ID NO: 3. In one embodiment of said method of identifying a microRNA which improves and/or increases the viral genome titre, capsid titre and/or potency of recombinant adeno-associated virus (rAAV) in a preparation produced during recombinant AAV production, said microRNA comprises or consists of SEQ ID NO: 3.

In one embodiment of said method of identifying a microRNA which improves and/or increases the viral genome titre, capsid titre and/or potency of recombinant adeno-associated virus (rAAV) in a preparation produced during recombinant AAV production, said microRNA has a sequence with at least 80% or at least 81%, or at least 82 %, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99% sequence identity with SEQ ID NO: 4. In one embodiment said of method of identifying a microRNA which improves and /or increases the the viral genome titre, capsid titre and/or potency of recombinant adeno-associated virus (rAAV) in a preparation produced during recombinant AAV production, said microRNA comprises or consists of SEQ ID NO: 4.

In one embodiment of said method of identifying a microRNA which improves and/or increases the viral genome titre, capsid titre and/or potency of recombinant adeno-associated virus (rAAV) in a preparation produced during recombinant AAV production, said first host cell line is a HEK293 cell line, preferably a suspension HEK293 cell line.

In one embodiment of said method of identifying a microRNA which improves and/or increases the viral genome titre, capsid titre and/or potency of recombinant adeno-associated virus (rAAV) produced during recombinant AAV production, said two plasmid system comprises a helper plasmid comprising at least one adeno-associated virus (AAV) rep gene encoding at least one functional AAV Rep protein and at least one helper virus gene, and which does not comprise a cap gene encoding a functional set of Cap proteins, and a vector plasmid comprising an AAV cap gene encoding at least one functional AAV Cap protein.

In one embodiment said AAV is a AAV S3 serotype.

In one embodiment said second cell line is a Huh7 cell line.

In an even further aspect, the disclosure relates to a use of a microRNA molecule in a method of identifying a microRNA which improves and/or increases the viral genome titre, capsid titre and/or potency of recombinant adeno-associated virus (rAAV) produced during recombinant AAV production, as described in the above embodiments referring to such a method.

Unless defined otherwise, technical and scientific terms used herein have the same meaning as commonly understood by a person skilled in the art to which this invention belongs. All publications, patent applications, patents and other references mentioned herein are incorporated by reference in their entirety.

In some embodiments, the term *"around"* or *"approximately"*in relation to a reference numerical value and its grammatical equivalents as used herein can include the numerical value itself and a range of values plus or minus 10% from that numerical value. For example, in these embodiments the term *"around* 10" includes 10 and any amounts from 9 to 11. For example, in one the term *"around"* in relation to a reference numerical value can also include a range of values plus or minus 10%, 9%, 8%, 7%, 6%, 5%, 4%, 3%, 2%, or 1% from that value.

In general, the term *"comprising"* is intended to mean including but not limited to. For example, the phrase "a *two plasmid system comprises a helper plasmid..."*should be interpreted to mean that the two plasmid system has at least a helper plasmid may comprise further components such as a further plasmid.

In some embodiments of the invention, the word *"comprising"* is replaced with the phrase *"consisting* of' or the phrase *"consisting essentially of".* The term *"consisting of"* is intended to be limiting.

The terms *"protein"* and *"polypeptide"* are used interchangeably herein and are intended to refer to a polymeric chain of amino acids of any length.

For the purpose of this invention, in order to determine the percent identity of two sequences (such as two polynucleotide or two polypeptide sequences), the sequences are aligned for optimal comparison purposes (*e.g.,* gaps can be introduced in a first sequence for optimal alignment with a second sequence). The nucleotides or amino acids at each position are then compared. When a position in the first sequence is occupied by the same amino acid or nucleotide as the corresponding position in the second sequence, then the amino acids or nucleotides are identical at that position. The percent identity between the two sequences is a function of the number of identical positions shared by the sequences (*i.e.,* % identity = number of identical positions /total number of positions in the reference sequence x 100).

Typically, the sequence comparison is carried out over the length of the reference sequence. For example, if the user wished to determine whether a given ("*test*") sequence is 95% identical to SEQ ID NO: 1, SEQ ID NO: 1 would be the reference sequence. To assess whether a sequence is at least 80% identical to SEQ ID NO: 1 (an example of a reference sequence), the skilled person would carry out an alignment over the length of SEQ ID NO: 1, and identify how many positions in the test sequence were identical to those of SEQ ID NO: 1. If at least 80% of the positions are identical, the test sequence is at least 80% identical to SEQ ID NO: 1. If the sequence is shorter than SEQ ID NO: 1, the gaps or missing positions should be considered to be non-identical positions.

The skilled person is aware of different computer programs that are available to determine the homology or identity between two sequences. For instance, a comparison of sequences and determination of percent identity between two sequences can be accomplished using a mathematical algorithm. In an embodiment, the percent identity between two amino acid or nucleic acid sequences is determined using the Needleman and Wunsch (1970) algorithm which has been incorporated into the GAP program in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6.

A "vector" as used herein refers to a macromolecule or association of macromolecules that comprises or associates with a polynucleotide and which can be used to mediate delivery of the polynucleotide to a cell. Illustrative vectors include, for example, plasmids, viral vectors, liposomes and other gene delivery vehicles.

"AAV" is an abbreviation for adeno-associated virus and may be used to refer to the virus itself or derivatives thereof. The term covers all subtypes and both naturally occurring and recombinant forms, except where required otherwise. The abbreviation "rAAV" refers to recombinant adeno-associated virus, also referred to as a recombinant AAV vector (or "rAAV vector").

An "rAAV vector" as used herein refers to an AAV vector comprising a polynucleotide sequence not of AAV origin (i.e., a polynucleotide heterologous to AAV), typically a sequence of interest for the genetic transformation of a cell. In preferred vector constructs of this invention, the heterologous polynucleotide is flanked by at least one, preferably two AAV inverted terminal repeat sequences (ITRs). The term rAAV vector encompasses both rAAV vector particles and rAAV vector plasmids.

An "AAV virus" or "AAV viral particle" refers to a viral particle composed of at least one AAV capsid protein (preferably by all of the capsid proteins of a wild-type AAV) and an encapsidated polynucleotide. If the particle comprises a heterologous polynucleotide (i.e. a polynucleotide other than a wild-type AAV genome such as a transgene to be delivered to a mammalian cell), it is typically referred to as an "rAAV vector particle" or simply an "rAAV vector".

"Packaging" refers to a series of intracellular events that result in the assembly and encapsidation of an AAV particle.

AAV "rep" and "cap" genes refer to polynucleotide sequences encoding replication and encapsidation proteins of adeno-associated virus. They have been found in all AAV serotypes examined and are in the art. AAV rep and cap are referred to herein as AAV "packaging genes".

A "helper virus" for AAV refers to a virus that allows AAV (e.g. wild-type AAV) to be replicated and packaged by a mammalian cell. A variety of such helper viruses for AAV are known in the art, including adenoviruses, herpesviruses and poxviruses such as vaccinia. The adenoviruses encompass a number of different subgroups, although Adenovirus type 5 of subgroup C is most commonly used. Numerous adenoviruses of human, non-human mammalian and avian origin are known and available from depositories such as the ATCC. Viruses of the herpes family include, for example, herpes simplex viruses (HSV) and Epstein-Barr viruses (EBV), as well as cytomegaloviruses (CMV) and pseudorabies viruses (PRV): which are also available from depositories such as ATCC.

Herein, the term *"plasmid"* is intended to refer to a nucleic acid molecule that can replicate independently of a cell chromosome. The term *"plasmid"* is intended to cover circular nucleic acid molecules and linear nucleic acid molecules. Furthermore, the term *"plasmid"* is intended to cover bacterial plasmids, but also cosmids, minicircles (Nehlsen, K., Broll S., Bode, J. (2006), Gene Ther. Mol. Biol., 10: 233-244; Kay, M.A., He, C.-Y, Chen, Z.-H. (2010), Nature Biotechnology, 28: 1287-1289) and ministrings (Nafissi N, Alqawlaq S, Lee EA, Foldvari M, Spagnuolo PA, Slavcev RA. (2014), Mol Ther Nucleic Acids, 3:e165). Optionally, the plasmid is a circular nucleic acid molecule. Optionally, the plasmid is a nucleic acid molecule that is of bacterial origin.

The term *"helper"* is not intended to be limiting. Accordingly, a *"helper plasmid"* is any plasmid that comprises at least one rep gene encoding at least one functional Rep protein and may or may not comprise a cap gene encoding a functional set of Cap proteins.

The term *"vector plasmid"* is not intended to be limiting. Accordingly, a *"vector plasmid"* is any plasmid that:
(i) is suitable for use alongside a helper plasmid in a two-plasmid system of the invention; and/or
(ii) comprises:
   (a) a cap gene encoding at least one functional Cap protein; or
   (b) at least one cap gene promoter, a cloning site operably linked to the cap gene promoter, and an expression cassette flanked on at least one side by an ITR;
wherein the vector plasmid does not comprise a rep gene encoding a functional Rep protein and the expression cassette comprises a transgene operably linked to at least one regulatory control element.

The term "three or *triple plasmid AAV production system"* refers to a rAAV production system which involves the transfection of host cells with three separate plasmids, namely a rAAV vector plasmid which contains a promoter, a gene of interest (GOI) flanked by inverted terminal repeats (ITRs), which are essential for AAV replication and packaging, a rAAV helper plasmid carrying the Rep and Cap genes, which encode the replication and capsid proteins of AAV, respectively and an adenovirus helper plasmid (Ad helper) containing the necessary adenoviral genes, such as, E2a, E4orf6, and VA RNA, which are required to support AAV replication and packaging. Currently, there are available several variations of the above-mentioned system in the art, which are herein incorporated by reference.

The term *"two plasmid AAV production system"* in the context of the current disclosure refers to a system that comprises only two plasmids and can be used without the need for additional plasmids to produce rAAV. Optionally, the two-plasmid system can be used to produce rAAV without the need for helper virus such as adenovirus. Optionally, the two plasmid system can be used to produce rAAV without the need for genetic material originating from a host cell, optionally with the exception of a gene encoding E1A/B. However, the system may comprise additional non-plasmid components. Optionally, the two plasmid system does not comprise a helper virus. Optionally, the two plasmid system of the invention comprises all the necessary genetic information for the production of rAAV. For example, the two plasmid system of the invention may comprise at least one rep gene, at least one cap gene and at least one helper gene. Optionally, the two plasmid system of the invention comprises all the necessary genetic information required for the production of rAAV suitable for use in gene therapy. For example, the two plasmid system of the invention may comprise at least one rep gene, at least one cap gene, at least one helper gene and an expression cassette comprising a transgene operably linked to at least one regulatory control element. However, in embodiments the two plasmid system of the invention may lack a functional cap gene (required for the production of rAAV) and/or an expression cassette comprising a transgene operably linked to at least one regulatory control element (required for the production of rAAV suitable for use in gene therapy). Suitably, a two plasmid system of the invention comprises a helper plasmid comprising at least one AAV rep gene encoding at least one functional AAV Rep protein and at least one helper virus gene, and which does not comprise a cap gene encoding a functional set of Cap proteins and a vector plasmid comprising (a) an AAV cap gene encoding at least one functional AAV Cap protein; or (b) at least one AAV cap gene promoter, a cloning site operably linked to the AAV cap gene promoter, and an expression cassette flanked on at least one side by an inverted terminal repeat (ITR); wherein the vector plasmid does not comprise a rep gene encoding a functional Rep protein and the expression cassette comprises a transgene operably linked to at least one regulatory control element. Suitable two plasmid systems are described in WO 2020/208379 A, EP3722434 B1 and WO 2022/079429 A1 (incorporated by reference).

It is an advantage of the present invention that a cap gene and/or a transgene in the vector plasmid may be exchanged with another in order to treat different genetic disorders. Optionally, therefore, the two plasmid system of the invention may comprise all the necessary genetic information for the production of rAAV except a functional cap gene, and in such embodiments the two plasmid system of the invention may comprise a site suitable for cloning in a cap gene. Such a site may comprise a cloning site adjacent to a cap gene promoter. The site suitable for cloning in a cap gene will be present on the vector plasmid. Optionally, the two plasmid system of the invention comprises all the necessary genetic information for the production of rAAV suitable for use in gene therapy except a functional cap gene and an expression cassette comprising a transgene and a regulatory control element,
The term *"nucleic acid molecule"* refers to a polymeric form of nucleotides of any length. The nucleotides may be deoxyribonucleotides, ribonucleotides or analogs thereof. Preferably, the plasmid is made up of deoxyribonucleotides or ribonucleotides. Even more preferably, the plasmid is made up of deoxyribonucleotides, *i.e.* the plasmid is a DNA molecule. In all instances herein, the term *"nucleic acid sequence"* may be replaced by the term *"polynucleotide".*

The terms *"wild type"* and *"native"* are synonymous and refer to genes present in the genome of a strain/serotype of AAV or adenovirus, or to proteins encoded by genes present in the genome of a strain/serotype of AAV or adenovirus.

The helper plasmid may be useful for producing rAAV. Optionally, the helper plasmid is suitable for use in producing rAAV. Optionally, the helper plasmid is for producing rAAV. Optionally, the helper plasmid is suitable for producing rAAV suitable for use in gene therapy. Optionally, the helper plasmid is for producing rAAV for use in gene therapy.

Transcription regulatory elements are nucleotide sequences which effect the level of expression of a gene, and include, for example, promoters, enhancers, introns, untranslated regions, and transcriptional terminators. Some transcription regulatory elements promote greater levels of transcription compared to others (stronger transcription regulatory elements). For example, some promoters are known to promote transcription at a higher level than others. Generally, a promoter will be a stronger promoter if it comprises a sequence that allows for strong binding to the transcription complex. Promoters which are known to be generally strong promoters in human cells include viral promoters. Promoters that are generally believed to be strong promoters in human cells include the EF1A, CMV, CAG and SV40 promoters. Promoters that are generally believed to be weak promoters in human cells include UBC and PGK promoters.

### Vector plasmid

As discussed above, the two plasmid system of the invention may comprise a vector plasmid.

### Cap gene

The vector plasmid may comprise a cap gene. The cap gene encodes a functional Cap protein. The cap gene may encode a functional set of Cap proteins. AAV generally comprise three Cap proteins, VP1, VP2 and VP3. These three proteins form a capsid into which the AAV genome is inserted and allow the transfer of the AAV genome into a host cell. All VP1, VP2 and VP3 are encoded in native AAV by a single gene, the cap gene. The amino acid sequence of VP1 comprises the sequence of VP2. The portion of VP1 which does not form part of VP2 is referred to as VP1unique or VP1U. The amino acid sequence of VP2 comprises the sequence of VP3. The portion of VP2 which does not form part of VP3 is referred to as VP2unique or VP2U.

A "functional" set of Cap proteins is one which allows for encapsidation of AAV. As discussed above, it is within the abilities of the skilled person to determine whether a given Cap protein is or a set of Cap proteins are functional. The skilled person merely needs to determine whether the encoded Cap protein(s) support AAV production using an AAV production assay as described above. In this case, the test two plasmid system will comprise a vector plasmid that comprises the cap gene which encodes the Cap protein(s) whose "functionality" is to be determined, and otherwise the test two plasmid system used will be identical to the reference two plasmid system. The Cap protein(s) will be considered to be "functional" if it/they support(s) rAAV production at a level at least 25%, at least 40%, at least 50%, at least 70%, at least 80%, at least 90% or at least 95% of the level supported by the wild type cap gene product, i.e. if the yield of rAAV produced is at least 25%, at least 40%, at least 50%, at least 70%, at least 80%, at least 90% or at least 95% of the yield of rAAV produced using the reference two plasmid system. Preferably, the Cap protein(s) will be considered to be "functional" if it/they support(s) rAAV production at a level at least 70%, at least 80%, at least 90% or at least 95% of the level supported by the wildtype cap gene product.

Optionally, VP2 and/or VP3 proteins are "functional" if an rAAV comprising the VP2 and/or the VP3 proteins is able to transduce Huh7 cells at a level at least 25%, at least 40%, at least 50%, at least 70%, at least 80%, at least 90% or at least 95% of that of an equivalent rAAV comprising a wild type VP2 and/or VP3 protein. The ability of an rAAV particle to transduce Huh7 cells can be tested applying a reporter gene encoding a reporter protein as rAAV transgene cassette, transducing Huh7 cells with the produced rAAV and measuring the fluorescence produced.

Optionally, the vector plasmid comprises a cap gene that encodes a VP1, a VP2 and/or a VP3 protein. Optionally, the VP1, VP2 and VP3 proteins are expressed from more than one cap gene. Optionally, the vector plasmid comprises a cap gene that encodes a VP1, a VP2 and a VP3 protein. Optionally the vector plasmid comprises a cap gene encoding a functional VP1, i.e. a VP1 protein capable of assembling with other Cap proteins to encapsidate a viral genome.

Different serotypes of AAV have Cap proteins having different amino acid sequences. A cap gene encoding any (set of) Cap protein(s) is suitable for use in connection with the present invention. The Cap protein can be a native Cap protein expressed in AAV of a certain serotype. Alternatively, the Cap protein can be a non-natural, for example an engineered, Cap protein, which is designed to comprise a sequence different to that of a native AAV Cap protein. Genes encoding non-natural Cap proteins are particularly advantageous, as in the context of gene therapy applications it is possible that fewer potential patients have levels of antibodies that prevent transduction by rAAV comprising non-natural Cap proteins, relative to native capsids.

Optionally, the cap gene encodes a Cap protein from a serotype selected from the group consisting of serotypes 1, 2, 3A, 3B, 4, 5, 6, 7, 8, 9, 10, 11, 12, or 13. Optionally, the cap gene encodes a Cap protein from a serotype selected from the group consisting of serotypes 2, 5, 8, and 9. Optionally, the cap gene encodes a Cap protein selected from the group consisting of LK03, rh74, rh10 and Mut C (WO 2016/181123; WO 2013/029030; WO 2017/096164). Optionally, the cap gene encodes a Cap protein selected from the group of AAV serotypes consisting of serotypes 2, 5, 8 or 9, and Mut C (SEQ ID NO: 3 from WO 2016/181123). Optionally, the cap gene encodes the Cap protein Mut C (SEQ ID NO: 3 from WO 2016/181123).

### Cap gene promoter

Optionally, the vector plasmid comprises a cap gene promoter. The cap gene promoter may be operably linked to a cap gene. Alternatively, the vector plasmid may not comprise a cap gene, but may comprise a cloning site operably linked (i.e. in close juxtaposition: 5'-[cap gene promoter]-[cloning site]-3') to the cap gene promoter. The user may wish to have the option to add a specific cap gene for a specific application. For example, if the vector plasmid is to be used to produce rAAV for use in gene therapy, the user may wish the vector plasmid to lack a cap gene but comprise a cloning site to allow a specific cap gene to be cloned in for a specific application. The vector plasmid could be used in connection with any transgene (in an expression cassette), and the user may find that for certain transgenes, encapsidation of such cassettes into capsids having properties such as liver tropism is advantageous, whereas for other transgenes capsids having different tropisms are advantageous. By designing a vector plasmid that comprises a cap gene promoter linked to a cloning site, the user can readily 'plug in' an appropriate cap gene for a specific application (such as use of a specific transgene).

Optionally, the vector plasmid comprises an at least one cap gene promoter, which is a native cap gene promoter.

The native cap gene (i.e. the cap gene of a wild type AAV) is operably linked to a p40 promoter, a p5 promoter and a p19 promoter. Optionally, the at least one cap gene promoter comprises an AAV p40 promoter, a p5 promoter, and/or a p19 promoter. Optionally, the at least one cap gene promoter comprises an AAV p40 promoter, a p5 promoter, and a p19 promoter. However, any suitable promoter that is able to drive cap gene expression can be used.

### Rep genes

A "functional" Rep protein is one which allows for production of AAV particles. In particular, Rep 78 or Rep 68 (the large Rep proteins) are believed to be involved in replication of the AAV genome, and Rep 52 and Rep 40 (the small Rep proteins) are believed to be involved in packaging of the AAV genome into a capsid. It is within the abilities of the skilled person to determine whether a given Rep protein is functional. The skilled person merely needs to determine whether the Rep protein supports AAV production using an AAV production assay as described above. In this case, the test two plasmid system will comprise a helper plasmid that comprises the Rep protein whose "functionality" is to be determined, and otherwise the test two plasmid system used will be identical to the reference twoplasmid system.

In an embodiment,the at least one rep gene of the helper plasmid encodes a "functional" Rep protein. If the Rep protein supports rAAV production at a level at least 25%, at least 40%, at least 50%, at least 70%, at least 80%, at least 90% or at least 95% of the level supported by the wild type Rep protein, i.e. if the yield of rAAV vector genomes produced is at least 25%, at least 40%, at least 50%, at least 70%, at least 80%, at least 90% or at least 95% of the yield of rAAV vector genomes produced using the reference two-plasmid system. Preferably, the at least one rep gene of the helper plasmid is functional if it supports rAAV production at a level at least 80% of the level supported by the wild type Rep protein.

In general, a Rep protein will only be able to support rAAV production if it is compatible with the ITR(s) surrounding the genome of the AAV to be packaged. Some Rep proteins may only be able to package genomic material (such as an expression cassette) when it is flanked by ITR(s) of the same serotype as the Rep protein. Other Rep proteins are cross-compatible, meaning that they can package genomic material that is flanked by ITR(s) of a different serotype. For example, in the case of a two-plasmid system, it is preferred that the Rep protein is able to support replication and packaging of an expression cassette comprised within the vector plasmid, and such a Rep protein will be compatible with the at least one ITR flanking the expression cassette (i.e. able to replicate and package the expression cassette flanked on at least one side by an ITR).

Optionally, the at least one rep gene comprises a gene encoding a functional Rep 52 protein, at least one gene encoding a functional Rep 40 protein, and a gene encoding a functional Rep 68 protein.

The helper plasmid may comprise two genes encoding a functional Rep 40 protein. In an embodiment, the at least one rep gene comprises two genes encoding a functional Rep 40 protein. For example, the helper plasmid may comprise two rep genes that are separated on the plasmid. The first of the two separate rep genes could encode Rep 68 (for example using the p5 promoter or a different promoter situated near the normal position of the p5 promoter in the rep gene) and Rep 40 (for example using the p19 promoter or a different promoter situated near the normal position of the p19 promoter). The second of the two separate rep genes could encode Rep 52 and Rep 40. Rep 52 and Rep 40 are alternative splice variants.

If the helper plasmid comprises two genes encoding a Rep 40 protein, one of the two genes that encodes a functional Rep 40 protein may comprise an intron. In one embodiment, both genes that encode a functional Rep 40 protein comprise an intron. However, in a preferred embodiment, only one of the genes that encodes a functional Rep 40 protein comprises an intron. For example, if the user wishes to avoid the at least one rep gene encoding Rep 78, the rep gene may be split through partial duplication into two genes. One gene could comprise nucleotides corresponding to the full-length native rep gene with the sequence corresponding to the intron removed. Such a gene would encode Rep 68 and Rep 40 but would not encode either Rep 78 or Rep 52, as a portion of each of the Rep 78 and Rep 52 proteins is encoded by the sequence which acts as an intron in the context of rep 40. The second gene could comprise nucleotides corresponding to the region of the native rep gene downstream of the p19 promoter, which would encode Rep 52 (intron spliced in) and Rep 40 (intron spliced out).

A two-plasmid system may be used in method of the invention to produce rAAV vector for use in gene therapy. A *"gene therapy"* involves administering rAAV/viral particles of the invention that are capable of expressing a transgene (such as a Factor IX-encoding nucleotide sequence) in the host to which it is administered. In such cases, the vector plasmid will comprise an expression cassette.

Optionally, the vector plasmid comprises at least one ITR. Thus, optionally, the vector plasmid comprises at least one ITR, but, more typically, two ITRs (generally with one either end of the expression cassette, i.e. one at the 5' end and one at the 3' end). There may be intervening sequences between the expression cassette and one or more of the ITRs. The expression cassette may be incorporated into a viral particle located between two regular ITRs or located on either side of an ITR engineered with two D regions. Optionally, the vector plasmid comprises ITR sequences which are derived from AAV1, AAV2, AAV4 and/or AAV6. Preferably the ITR sequences are AAV2 ITR sequences.

Optionally, the vector plasmid comprises an expression cassette. As described herein, an expression cassette refers to a sequence of nucleic acids comprising a transgene and a promoter operably linked to the transgene. Optionally, the cassette further comprises additional transcription regulatory elements, such as enhancers, introns, untranslated regions, transcriptional terminators, etc.

The expression cassette should be considered to comprise the stretch of the vector plasmid between the ITRs that comprises any transgenes and transcription regulatory elements operably linked to the transgene. In embodiments where the vector plasmid comprises two ITRs, the expression cassette is considered to comprise the stretch of the vector plasmid between and including the ITRs. Optionally, the expression cassette is less than 5.0 kbp, less than 4.9 kbp, less than 4.8 kbp, less than 4.75 kbp, less than 4.7 kbp, or less than 4.5 kbp. Optionally, the expression cassette is less than 4.7kbp, less than 4.6kbp, less than 4.5kbp, less than 4.4 kbp, less than 4.3 kbp, less than 4.2 kbp, less than 4.1 kbp, less than 4.0 kbp, less than 3.9 kbp, less than 3.8 kbp, less than 3.7 kbp, less than 3.6 kbp, less than 3.5 kbp, less than 3.4 kbp, less than 3.3 kbp, less than 3.2 kbp, less than 3.1 kbp, less than 3.0 kbp, less than 2.9 kbp, less than 2.8 kbp, less than 2.7 kbp, less than 2.6 kbp, less than 2.5 kbp, less than 2.4 kbp, less than 2.3 kbp, less than 2.2 kbp, less than 2.1 kbp, less than 2.0 kbp, 1.9 kbp, less than 1.8 kbp, less than 1.7 kbp, less than 1.6 kbp, less than 1.5 kbp, less than 1.4 kbp, less than 1.3 kbp, less than 1.2 kbp, less than 1.1 kbp, less than 1.0 kbp, less than 0.9 kbp, less than 0.8 kbp, less than 0.7 kbp, less than 0.6 kbp, less than 0.5 kbp, less than 0.4 kbp, less than 0.3 kbp, less than 0.2 kbp, or less than 0.1 kbp. Optionally, the expression cassette is between less than 4.9kb and between less than 4.7kb. Optionally, the expression cassette is between 2.0 kbp and 5.5 kbp, between 2.0 kbp and 4.75 kbp, between 2.0 kbp and 4.7 kbp, between 2.1 kbp and 4.9 kbp, between 2.2 kbp and 4.8 kbp or between 2.3 kbp and 4.5 kbp.

Optionally, the expression cassette comprises a transcription regulatory element comprising the promoter element and/or enhancer element from HLP2, HLP1, LP1, HCR-hAAT, ApoE-hAAT, and/or LSP. These transcription regulatory elements are described in more detail in the following references: HLP2: WO16/075473; HLP1: McIntosh J. et al., Blood 2013 Apr 25, 121(17):3335-44; LP1: Nathwani et al., Blood. 2006 April 1, 107(7): 2653-2661; HCR-hAAT: Miao et al., Mol Ther. 2000;1: 522-532; ApoE-hAAT: Okuyama et al., Human Gene Therapy, 7, 637-645 (1996); and LSP: Wang et al., Proc Natl Acad Sci USA. 1999 March 30, 96(7): 3906-3910. Each of these transcription regulatory elements comprises a promoter, an enhancer, and optionally other nucleotides. If the polynucleotide is intended for expression in the liver, the promoter may be a liver-specific promoter. Optionally, the promoter is a human liver-specific promoter.

The transgene may be any suitable gene. If the vector plasmid is for use in gene therapy, the transgene may be any gene that comprises or encodes a protein or nucleotide sequence that can be used to treat a disease. For example, the transgene may encode an enzyme, a metabolic protein, a signalling protein, an antibody, an antibody fragment, an antibody-like protein, an antigen, or a non-translated RNA such as a miRNA, siRNA, snRNA, or antisense RNA.

Optionally, the vector plasmid comprises a cap gene and further comprises an expression cassette flanked on at least one side by an ITR.

### Host cell line

The present invention provides a host cell line.

The host cell line is preferably a host cell line that can be used to produce rAAV. The host cell line may therefore be a host cell line suitable for the production of rAAV. In addition, the host cell line may be for the production of rAAV.

In general, a host cell line that is suitable for the production of rAAV is a host cell line that is derived from a eukaryotic cell line, preferably a vertebrate cell line, preferably a mammalian cell line, preferably a human cell line. Optionally, the host cell is a cell selected from the group consisting of a HEK293T cell, a HEK293 cell, a HEK293EBNA cell, a HEK293F cell, aHEK293S cell, a CAP cell, a CAP-T cell, an AGE1.CR cell, a PerC6 cell, a C139 cell, an EB66 cell, a BHK cell, a COS cell, a Vero cell, a HeLa cell, and an A549 cell. Preferably, the host cell is selected from the group consisting of a HEK293T cell, a HEK293 cell, a HEK293EBNA cell, a CAP cell, a CAP-T cell, an AGE1.CR cell, a PerC6 cell, a C139 cell, and an EB66 cell. Even more preferably, the host cell is selected from the group consisting of a HEK293T cell, a HEK293 cell, and a HEK293EBNA cell. For example, the host cell may be a HEK293T cell or a HEK293 cell. Optionally, the host cell is a cell that expresses a functional adenoviral E1A/B protein.

It is within the abilities of the skilled person to determine whether a host cell is suitable for the production of rAAV. The skilled person merely needs to determine whether the host cell supports rAAV production using an rAAV production assay as described above. In this case, the test two-plasmid system and the reference two-plasmid system that are used will be identical. However, the test two-plasmid system will be transfected into the host cell whose suitability for the production of recombinant AAV is to be tested, and the reference two-plasmid system will be transfected into HEK293T cells. The host cell will be considered to be suitable for the production of recombinant AAV if it supports AAV production at a level at least 30%, at least 40%, at least 50%, at least 70%, at least 80%, at least 90%, or at least 95% of the level supported by HEK293T cells, i.e. if the yield of recombinant AAV produced is at least 30%, at least 40%, at least 50%, at least 70%, at least 80%, at least 90%, or at least 95% of the yield of recombinant AAV produced in HEK293T cells.

In the context of the present disclosure, the term "host cell" and "host cell line" are used interchangeably.

### High (vector genome) yield

The term *"yield"* refers to the amount of rAAV particles that are prepared in the methods or uses of the invention. The *"yield"* may be expressed as the number of vector genomes (vg) per ml of medium, as measured on bulk product prior to harvesting, purifying and/or concentrating the rAAV preparation. The yield of rAAV (such as rAAV particles) may be determined by using qPCR to quantify the number of nucleic acid sequences comprising a cassette comprising a promoter sequence (vg).

As used herein the term *"high yield"* generally refers to a yield at least 1.05 fold greater than the yield achieved using an equivalent or corresponding method. without prior introduction of the host cell with said same microRNA molecule or said same microRNA expressing vector of the invention.

As used herein the term *"maximising"* the yield refers to aiming for the highest possible yield within the limitations of the methods of the invention. As used herein the term *"optimising"* refers to increasing the yield, but aiming for a desired yield which may not be the maximum possible yield if, for example, the user is keen to ensure a high ratio of full to total particles (i.e. minimising the proportion of empty particles).

Optionally, the higher yield is at least 1.05 fold, at least 1.1 fold, at least 1.2 fold, at least 1.25 fold, at least 1.5 fold, at least 1.6 fold, at least 1.7 fold, at least 2 fold, between 1.1 fold and 5 fold, between 1.2 fold and 3 fold, or between 1.2 fold and 2.5 fold higher than the yield obtained using an equivalent method.

### Increased viral genome titre

The methods of the disclosure may result in a preparation that comprises recombinant AAV at an improved, increased or higher viral genome titre. Viral genome titre is the concentration of viral genome particles present in a preparation. If the preparation comprises rAAV, the viral genome particles will be AAV viral genome particles. Viral genome titre can be used as a measure of the yield of rAAV. A preparation comprising recombinant AAV produced by the methods of the disclosure may have an improved, increased or higher viral genome titre when compared to a preparation comprising recombinant AAV produced by a corresponding method, such as a corresponding method in which the host cell is not introduced with an miRNA or miRNA expressing vector of the invention. In one embodiment, the method according to the invention produces a preparation comprising recombinant AAV having an improved, increased or higher viral genome titre. The preparation comprising recombinant AAV is the preparation that is produced by the methods of the invention. Accordingly, to determine whether a method produces a preparation comprising recombinant AAV having or an improved, increased or higher viral genome titre, the viral genome titre of the preparation should be measured once the method is completed and if the method or use comprises further subsequent steps once those further subsequent steps have been completed, and compared to the viral genome titre of a preparation made using an equivalent method in which the host cell is not introduced with the same miRNA or miRNA expressing vector.

In another embodiment, the method according to the invention produces a preparation comprising recombinant AAV having at least 10%, at least 20%, at least 30%, at least 40%, at least 50%, or at least 60% improved, increased or higher viral genome titre when compared to a preparation comprising recombinant AAV produced by a corresponding method in which the host cell is not introduced with the same miRNA or miRNA expressing vector.

In another embodiment, the method according to the invention is a method for improving or increasing the viral genome titre of a preparation comprising recombinant AAV.

In another embodiment, the method according to the invention is a method for improving or increasing the viral genome titre of a preparation comprising recombinant AAV by at least 10%, at least 20%, at least 30%, at least 40%, at least 50%, or at least 60%, at least 70%, or at least 80%, when compared to a preparation comprising recombinant AAV produced by a corresponding method in which the host cell is not is not introduced with the same miRNA or miRNA expressing vector.. Alternatively, the viral genome titre is at least 1.05 fold, at least 1.1 fold, at least 1.2 fold, at least 1.25 fold, at least 1.5 fold, at least 1.6 fold, at least 1.7 fold, at least 2 fold, between 1.1 fold and 5 fold, between 1.2 fold and 3 fold, or between 1.2 fold and 2.5 fold higher than the viral genome titre obtained using an equivalent method.

### Viral genome titre assay

The skilled person will understand that there are many suitable methods for determining the viral genome titre of a preparation comprising recombinant AAV. Viral genome titre may be measured using digital droplet polymerase chain reaction (ddPCR). Viral genome titre may be measured by the quantitative polymerase chain reaction (qPCR). qPCR or ddPCR may be carried out with primers specific to the viral genome. For example, if the viral genome is the AAV genome, primers specific to the AAV genome will be used. Viral genome titre may be measured using photometric quantification.

The AAV viral genome assay may be based on a quantitative polymerase chain reaction (qPCR) specific for the promoter sequence of the rAAV expression cassette. In principle, the qPCR primers can be designed to bind any part of the recombinant AAV genome which is not common to wild type AAV genomes, but it is recommended against using primer template sequences very close to the ITRs as doing so can lead to an exaggerated vector genome titre measurement. Suitably, qPCR is carried out using a pair of primers that are able to amplify at least a region of the promoter of the expression cassette. Optionally, at least one of the primers is specific for (reverse and complementary to or identical to depending on whether the primer is a forward primer or a reverse primer) a region of at least 12, at least 14, at least 16, or at least 18 nucleotides of the promoter of the expression cassette. Optionally, one primer is specific for the start of the promoter (the first at least 12 nucleotides of the promoter) and the other primer is specific for a region of the expression cassette that is 150 base pairs from the binding site of the first primer. The qPCR may be performed using SYBR green or another intercalating dye that can be used for detection of the amplification product. Alternatively, the qPCR product may be detected using TaqmanTM assay or similar.

Cell lysate test samples may be subjected to a nuclease treatment procedure in order to remove non-packed vector genomes prior to performing qPCR or ddPCR.

"Droplet digital PCR" (ddPCR) refers to a digital PCR assay that measures absolute quantities by counting nucleic acid molecules encapsulated in discrete, volumetrically defined, water-in-oil droplet partitions that support PCR amplification (i.e., a plurality of such compartments). Typically, a "droplet" refers to water-in-oil droplet (i.e., an oil droplet that may be generated by emulsifying a sample with droplet generator oil); an individual partition of the droplet digital PCR assay. A droplet supports PCR amplification of template molecule(s) using homogenous assay chemistries and workflows similar to those widely used for real-time PCR applications (Hinson et al (2011) Anal. Chem. 83:8604-8610; Pinheiro et al (2012) Anal. Chem. 84:1003-1011). A single ddPCR reaction may typically be comprised of at least 5000, at least 6000, at least 7000, at least 8000, at least 9000, at least 10,000, at least 12,000, at least 14,000, at least 16,000, at least 18,000 or at least 20,000 compartments.

Droplet digital PCR may be performed using any platform that performs a digital PCR assay that measures absolute quantities by counting nucleic acid molecules encapsulated in discrete, volumetrically defined, water-in-oil droplet partitions that support PCR amplification. The strategy for droplet digital PCR may be summarized as follows: a sample is diluted and partitioned into thousands to millions of separate reaction chambers (water-in-oil droplets) so that each contains one or no copies of the nucleic acid molecule of interest. The number of positive droplets detected, which contain the target amplicon (i.e., nucleic acid molecule of interest), versus the number of negative droplets, which do not contain the target amplicon (i.e., nucleic acid molecule of interest), may be used to determine the number of copies of the nucleic acid molecule of interest that were in the original sample. Examples of droplet digital PCR systems include the QX100^{™} Droplet Digital PCR System by Bio-Rad, which partitions samples containing nucleic acid template into 20,000 nanolitre-sized droplets.

Droplet digital PCR may thus be used to detect a single target in a sample, for example using a single primer pair. However, ddPCR may also be used to detect two different targets in a sample, for example using two primer pairs, each primer pair hybridising to a different target, i.e., duplexing of targets. Duplexing may be extended to look at more targets in a sample, such as at least 3, at least 4, at least 5, at least 6, at least 7, at least 8, at least 9, at least 10, at least 11 or at least 12 different targets in a sample, i.e., multiplexing of targets. Duplexing and multiplexing with ddPCR allows for improved sensitivity and precision, increased low level detection, and also the inference of the size of a nucleic acid. ddPCR may be quantitative.

### Increased/Improvedcapsid titre

Capsid titre is the concentration of capsid particles present in a preparation. For example, if the preparation comprises rAAV, the capsid titre is the concentration of AAV capsid particles in the preparation. The preparation comprising recombinant AAV produced when a host cell is introduced with an miRNA or miRNA expressing vector of the invention may have increased or improved capsid titre when compared to a preparation comprising recombinant AAV produced by a corresponding method in which the host cell is not introduced with the same miRNA or miRNA expressing vector. In one embodiment, the method according to the invention produces a preparation comprising recombinant AAV having increased or improved capsid titre. The preparation comprising recombinant AAV is the preparation that is produced by the methods.

In another embodiment, the method or use according to the invention produces a preparation comprising recombinant AAV having at least 10%, at least 20%, at least 30%, at least 40%, at least 50%, at least 60%, at least 70%, or at least 80% increased or improved capsid titre when compared to a preparation comprising recombinant AAV produced by a corresponding method in which the host cell is nott introduced with the same miRNA or miRNA expressing vector..

In another embodiment, the method or use according to the invention is a method or use for increasing the capsid titre of a preparation comprising recombinant AAV.

In another embodiment, the method or use according to the invention is a method or use for increasing the capsid titre of a preparation comprising recombinant AAV by at least 10%, at least 20%, at least 30%, at least 40%, at least 50%, at least 60%, at least 70%, or at least 80%, when compared to a preparation comprising recombinant AAV produced by a corresponding method in which the host cell is is not introduced with the same miRNA or miRNA expressing vector.. Alternatively, the capsid titre is at least 1.05 fold, at least 1.1 fold, at least 1.2 fold, at least 1.25 fold, at least 1.5 fold, at least 1.6 fold, at least 1.7 fold, at least 2 fold, between 1.1 fold and 5 fold, between 1.2 fold and 3 fold, or between 1.2 fold and 2.5 fold higher than the capsid titre obtained using an equivalent method.

In the context of the present disclosure an "improved and/or increased capsid titre" is used as an indication of "improved and/or increased genome titre".

### Capsid titre assay

The skilled person will understand that there are many suitable methods for determining the capsid titre of a preparation comprising recombinant AAV. Capsid titre may be measured by an enzyme-linked immunosorbent assay (ELISA). For example, the capsid-specific ELISA may comprise exposing the rAAV preparation to an antibody that binds to the capsid protein. If, for example, the vector plasmid comprises a cap gene that encodes a capsid from an AAV S3 serotype, the antibody may be an antibody that binds to the AAV S3 capsid. For example, the user may coat a plate with an antibody that is specific for the capsid. The user may then pass the rAAV preparation over the surface of the plate. The capsids will bind to the antibody and be immobilised on the plate. The plate may then be washed to remove contaminants. The amount of capsids present can then be detected by addition of a detection antibody that can bind to the capsid and is conjugated to a detection agent such as streptavidin peroxidase. The amount of capsids present will be proportional to the colour change obtained when the streptavidin peroxidase is exposed to the chromogenic substrate TMB (tetramethylbenzidine). In one aspect of the present invention, the capsid titre is measured by ELISA.

*Increasing the potency of recombinant AAV produced during recombinant AAV production.*

The present disclosure provides a method for increasing the potency of recombinant AAV produced during recombinant AAV production.

The term "potency" refers to the ability of the recombinant AAV to transduce cells and deliver a transgene. The "potency" may be measured by transducing cells with recombinant AAV produced using the methods of the invention which comprise a transgene and determining the activity of a polypeptide encoded by the transgene (i.e. a cell-based transduction assay). For example, a user may determine the potency of recombinant AAV produced by a particular method by carrying out the particular method of the invention comprising a transgene whose activity can be measured. The user may then measure the potency of the recombinant AAV by transducing host cells, such as Huh7 cells, with the recombinant AAV (for example at a pre-selected multiplicity of infection) and culturing the host cell under conditions suitable for expression of the transgene to occur. The user may then isolate the protein encoded by the transgene and test its activity. The potency assay may be an absolute potency assay.

Optionally, "potency" of recombinant AAV is measured by transducing host cells with the recombinant AAV, culturing the host cells and measuring the activity of a polypeptide produced by the host cells, wherein the recombinant AAV comprise a transgene and the polypeptide is encoded by the transgene. Optionally, potency of recombinant AAV is measured by transducing Huh7 cells with the recombinant AAV, culturing the Huh7 cells and measuring the activity of a polypeptide produced by the host cells, wherein the recombinant AAV comprise a transgene and the polypeptide is encoded by the transgene.

The present invention also provides a method for producing a recombinant AAV preparation having higher potency than the potency obtained using an equivalent method in which the host cell is not introduced with the same miRNA or miRNA expressing vector.

Optionally, the higher potency is at least 1.05 fold, at least 1.15 fold, between 1.1 fold and 5 fold, or between 1.15 fold and 3 fold higher than the potency of recombinant AAV obtained using said equivalent method. Optionally, the higher potency is between 1.15 fold and 3 fold higher than the potency of recombinant AAV obtained using said equivalent method. Optionally, the higher potency is at least 10%, at least 20%, at least 30%, at least 40%, at least 50%, at least 60%, at least 70% or at least 80% higher than the potency of recombinant AAV obtained using said equivalent method.

The method may further comprise a step of harvesting the rAAV vector to provide an rAAV preparation, optionally wherein the recombinant AAV preparation comprises a high or desired yield of recombinant AAV or comprises a yield that is higher than the yield obtained using an equivalent method or use using a two-plasmid system comprising a rep 78 negative helper plasmid. As set out above, the *"yield"* may be expressed as the number of vector genomes (vg) per ml of medium, as measured on bulk product prior to harvesting, purifying and/or concentrating the rAAV preparation. Thus, the *"recombinant AAV preparation comprises a high yield or desired yield of recombinantAA V'* if the bulk AAV product produced by the method or use is at a high yield or desired yield of recombinant AAV.

### High capsid yield

The term *"capsid yield'* refers to the amount of rAAV capsids that are prepared in the methods or uses of the invention. The *"capsid yield"* may be expressed as the number of capsids per ml of medium (as measured on bulk product prior to harvesting, purifying and/or concentrating the rAAV preparation).

As used herein, the term *"high capsid yield'* generally refers to a capsid yield that is at least or 1.05 or 1.1 or 1.2 fold higher than the capsid yield achieved, using an equivalent or corresponding method without using said same synthetic microRNA molecule or said same microRNA expressing vector.

An *"equivalent method"* or *"corresponding method"* is a method that is identical, except that said same synthetic microRNA molecule or said same microRNA expressing vector is not introduced to the host cell or isnot used in transfecting the host cell and wherein a comparative control may be used, such as the control(s) described in the examples section.

### Nucleic acid impurities

The term *"nucleic acid impurities"* refers to genetic material that has been packaged into rAAV and which was not intended to be packaged. For example, if the vector plasmid comprises two ITRs, the genetic material which was intended to be packaged into the rAAV is any genetic material between the two ITRs of the vector plasmid, i.e. the *"nucleic acid impurities"* is any genetic material that is not the genetic material between the two ITRs. The level of nucleic acid impurities may be as measured on bulk product prior to harvesting, purifying and/or concentrating the rAAV preparation, or may be measured after the rAAV have been harvested, purified and/or concentrated.

The level of nucleic acid impurities may be measured using a similar method to the qPCR method for quantifying the number of vector genomes but using qPCR primers specific for the nucleic acid impurities of interest. This qPCR method will provide the copy number of the nucleic acid impurities of interest per ml. The level of nucleic acid impurities may be expressed as percentage of the copy number of vector genomes per ml (as determined using the qPCR method for quantifying the number of vector genomes), i.e. the percentage/level of recombinant AAV comprising nucleic acid impurities may be expressed as the copy number of nucleic acid impurities of interest per ml / the copy number of vector genomes (vg) per ml x 100. When the level of nucleic acid impurities is expressed as a percentage of the copy number of vector genomes, it will be considered to be normalised to vector genome level.

The method may further comprise a step of harvesting the rAAV vector to provide an rAAV preparation, optionally wherein the recombinant AAV of the recombinant AAV preparation have a high potency or a potency that is higher than the potency of recombinant AAV produced using an equivalent method as described above.

*"Full"* particles are rAAV particles comprising both a capsid and the intended vector genome, or at least a partial such genome as determined using the qPCR method described below. *"Empty"* particles (i.e. particles which are not full) comprise capsids but do not comprise a genome or comprise only a partial genome (thereby not forming a complete rAAV particle) which is not detected using the qPCR method. However, the rAAV preparations may comprise both full particles and empty particles. Generally, a low or minimised proportion of empty particles is desired. For example, if the rAAV are to be used in gene therapy, any empty particles will not comprise the entire expression cassette of interest and so will not be effective in therapy. On the other hand, there are circumstances where the presence of empty particles could be desirable. In some instances, and in some patient groups, it may be the case that the empty particles behave as *"decoys"* to reduce the immune response in a patient to the administered rAAV particles (WO2013/078400).

The ratio of full to total particles may be expressed herein as the percentage of the total number of particles (capsids) that notionally comprise a vector genome or at least a partial such genome (assuming one (partial) genome per capsid) as determined using the following qPCR assay. qPCR is carried out using a pair of primers that are able to amplify at least a region of the promoter of the expression cassette. Optionally, at least one of the primers is specific for (reverse and complementary to or identical to depending on whether the primer is a forward primer or a reverse primer) a region of at least 12, at least 14, at least 16, or at least 18 nucleotides of the promoter of the expression cassette. Optionally, one primer is specific for the start of the promoter (the first at least 12 nucleotides of the promoter) and the other primer is specific for a region of the expression cassette that is 150 base pairs from the binding site of the first primer.

The ratio of full to total particles (as measured as a percentage of the total number of particles that are full particles) may be determined using qPCR to determine the number of vector genomes (as discussed in the previous paragraph) and using a capsid-specific immunoassay to measure the total number of particles. For example, the capsid-specific ELISA may comprise exposing the rAAV preparation to an antibody that binds to the capsid protein. If, for example, the vector plasmid comprises a cap gene that encodes a capsid from an AAV3 serotype, the antibody may be an antibody that binds to the AAV3 capsid. For example, the user may coat a plate with an antibody that is specific for the capsid. The user may then pass the rAAV preparation over the surface of the plate. The particles will bind to the antibody and be immobilised on the plate. The plate may then be washed to remove contaminants. The amount of particle present can then be detected by addition of a detection antibody that can bind to the capsid and is conjugated to a detection agent such as streptavidin peroxidase. The amount of particle present will be proportional to the colour change obtained when the streptavidin peroxidase is exposed to the chromogenic substrate TMB (tetramethylbenzidine).

Optionally, the desired ratio of full to total particles (expressed as a percentage of the total number of particles that notionally comprise a vector genome) is at least 2.5%, at least 5%, at least 7.5%, at least 8%, between 2.5% and 10%, between 5% and 9%, between 7.5% and 9%, or between 8% and 9% (as measured on bulk product prior to harvesting, purifying and/or concentrating the rAAV preparation). In many cases, increasing the full to total particle ratio is advantageous, and the present inventors have determined that aiming for a full to total particle ratio that is at least 2.5%, at least 5%, at least 7.5%, at least 8%, between 2.5% and 10%, between 5% and 9%, between 7.5% and 9%, or between 8% and 9% achieves a good balance between maintaining a high full to total particle ratio, whilst also achieving a good yield. Optionally, the desired ratio of full to total particles is a ratio of full to total particles that is at least 20% or at least 30% of the ratio of full to total particles achieved using an equivalent method.

In the context of the present disclosure "a *method for improving the viral genome titre, capsid titre and*/*or potency of recombinant adeno-associated virus (rAA V) produced during recombinant AA V production"* may refer to a method which confers a positive effect on the quality features of the produced viral genome titre, capsid titre and/or potency of recombinant adeno-associated virus (rAAV). Such quality features include, but are not limited to, reduced host cell DNA impurities/packaging, reduced packaging of non AAV vector genome DNA sequences like host cell DNA sequences/impurities and/or plasmid-derived DNA sequences/impurities, reduced empty to full capsid ratio, overall increased viral genome integrity, reduced aggregate content etc.

### Transfecting, culturing and harvesting

The methods of the invention may comprise steps of obtaining the two-plasmid system or the helper plasmid of the invention, transfecting a host cell with the two-plasmid system, or the helper plasmid, and culturing the host cell under conditions suitable for recombinant AAV production.

Transfecting a host cell with the two-plasmid system, or the helper plasmid, may comprise exposing the host cell to the two-plasmid system, or the helper plasmid in conditions suitable for transfection. For example, the user of the method may add a transfection agent (addition of a transfection agent would be considered to be a condition suitable for transfection). Alternatively, calcium phosphate transfection, electroporation or cationic liposomes could be used.

Culturing the host cell under conditions suitable for rAAV production refers to culturing the host cell under conditions at which it can grow and AAV can replicate. For example, the host cell may be cultured at a temperature between 32°C and 40°C, between 34°C and 38°C, between 35°C and 38°C, or around 37°C. Optionally, the host cell may be cultured in the presence of a complete cell culture medium such as BalanCD HEK293 medium supplemented with 4 mM L-Glutamine.. A complete cell culture medium is a medium that provides all the essential nutrients required for growth of the host cell. Optionally, the complete cell culture medium is supplemented with serum, such as fetal bovine serum or bovine serum albumin.

Optionally, the host cell is a host cell such as those defined above under the heading *"host cell".* Optionally, the host cell is selected from the group consisting of a HEK293T cell, a HEK293 cell, a HEK293EBNA cell, a CAP cell, a CAP-T cell, an AGE1.CR cell, a PerC6 cell, a C139 cell, and an EB66 cell. Optionally, the host cell is a cell that expresses a functional E1A/B protein.

The two-plasmid system may be used to produce rAAV in any suitable cell culture system. Optionally, culturing the host cell under conditions suitable for rAAV production comprises culturing the host cell using a suspension or an adherent system. Optionally, culturing the host cell under conditions suitable for rAAV production comprise culturing the host cell using a suspension system.

For the purposes of the present invention, the term *"suspension system"* refers to a system suitable for suspension cell culture, i.e. a system which allows cells to grow free-floating in culture medium. Cells in a suspension system may form aggregates or may be suspended in medium as single cells. For the purposes of the present invention, the term *"adherent system"* refers to a system suitable for adherent cell culture, i.e. for cells to be cultured whilst anchored to a substrate. Optionally, an adherent system refers to a flask or fermenter which forms a container to which cells can bind, and optionally is specifically treated to allow cell adhesion and spreading. Alternatively, an adherent system may be a *"carrier system",* in which the container contains an additional carrier such as a bead or a fibre to which the cells can adhere. In such *"carrier"* adherent systems, the cells tend to adhere less tightly and to have a morphology that is more similar to the morphology of cells grown using suspension systems compared to cells grown in conventional adherent systems.

The method may further comprise a step of purifying the rAAV. In general, a step of purifying the rAAV will involve increasing the concentration of the rAAV compared to other components of the preparation. Optionally, the step of purifying the rAAV results in a concentrated rAAV preparation. Optionally, the step of purifying the rAAV results in an isolated rAAV.

Any suitable purification method may be used. Optionally, the step of purifying the rAAV comprises a technique selected from the group consisting of gradient density centrifugation (such as CsCI or lodixanol gradient density centrifugation), filtration, ion exchange chromatography, size exclusion chromatography, affinity chromatography and hydrophobic interaction chromatography. Optionally, the step of purifying the rAAV is carried out using a technique selected from the group consisting of gradient density centrifugation (such as CsCI or lodixanol gradient density centrifugation), filtration, ion exchange chromatography, size exclusion chromatography, affinity chromatography and hydrophobic interaction chromatography.

Optionally, the method comprises further concentrating the rAAV using ultracentrifugation, tangential flow filtration, or gel filtration.

Optionally, the method comprises formulating the rAAV with a pharmaceutically acceptable excipient. The pharmaceutically acceptable excipients may comprise carriers, diluents and/or other medicinal agents, pharmaceutical agents or adjuvants, etc. Optionally, the pharmaceutically acceptable excipients comprise saline solution. Optionally, the pharmaceutically acceptable excipients comprise human serum albumin.

### EXAMPLES

### General techniques

The practice of the present invention will employ, unless otherwise indicated, conventional techniques of molecular biology, virology, animal cell culture and biochemistry which are within the skill of the art. Such techniques are explained fully in the literature. See, for example, "Molecular Cloning: A Laboratory Manual", Second Edition (Sambrook, Fritsch & Maniatis, 1989); "Animal Cell Culture" (R.I. Freshney, ed., 1987); "Gene Transfer Vectors for Mammalian Cells" (J.M. Miller & M.P. Calos, eds., 1987); "Current Protocols in Molecular Biology" (F.M. Ausubel et al., eds., 1987); "Current Protocols in Protein Science" (John E Coligan, et al. eds. Wiley and Sons, 1995); and "Protein Purification: Principles and Practice" (Robert K. Scopes, Springer-Verlag, 1994).

All patents, patent applications, articles and publications mentioned herein, both supra and infra, are hereby incorporated herein by reference.

### Example 1: High-throughput screening process of a miRNA library to identify miRNAs which improve and/or increase AAV production

In order to identify miRNA molecules which potentially improve and/or increase rAAV production yield, the Human miRIDIAN miRNA Mimic Library (Horizon Discovery) containing every human miRNA in the miRBase sequence database (version 21.0) was used for the miRNA screening process. This library comprises 2603 miRNA mimics, distributed on 33 96-well plates (so-called Master Plates (MP)), with approximately 80 miRNA mimics each. The miRNA screen was performed with biological triplicates applying data normalization against the respective on-plate non targeting siRNA (NT-siRNA) control to allow for inter-plate comparisons. The screening process involved the following steps.

### A. Experimental setup

### i) RNA transfection of HEK293 suspension cells in the 96-well format during the miRNA screen

Transient RNA transfections of HEK293 suspension cells for the miRNA screen were performed on suspension F-bottom 96-well plates (Greiner Bio-One GmbH). For that reason, a non-targeting siRNA control (NT-siRNA) and a AAV capsid specific siRNA control (cap_1990) were used, diluted in nuclease-free water to reach a final concentration of 100 nM per well. The non- targeting siRNA NT-siRNA (purchased by Qiagen) was used for normalization since it has no homology to any known mammalian gene and enables a clear differentiation between specific and non-specific effects of miRNA mimics on rAAV production. The cap_1990 siRNA was designed as rAAV-specific negative trending control using state-of-the-art siRNA design software. This siRNA targets VP1, VP2 and VP3 and was named according to its starting position within the AAVS3 capsid encoding sequence.

miRNA mimics from the miRNA mimics library (96-well plate format, 0.25 nmol; Horizon Discovery) were resuspended in nuclease-free water to reach a final concentration of 100 nM per well. 5 µL of controls and miRNA mimics were distributed in triplicates to the 96-well plates. 0.2 µL Lipofectamine^{®} 3000 (Invitrogen) per well was diluted in pre-warmed BalanCD HEK293 medium (Irvine Scientific) supplemented with 4 mM L-Glutamine to reach a final volume of 5 µL per well. 5 µL RNA transfection reagent mix per well was added to the diluted RNA and incubated for 10-15 min at room temperature.

HEK293 suspension cells in BalanCD HEK293 medium supplemented with 4 mM L-Glutamine coming from a 3-day culture with a seeding density of 1.35E+04 vc in 90 µL were added per well (CEDEX SX Cell Analyzer, Roche). The 96-well plates were covered with a BreathSeal (Greiner Bio-One GmbH) in addition to the plate lid and incubated at 37°C, 5% CO₂, 600 rpm on an orbital shaker with a 3 mm orbit (VWR) for 48h. After this incubation time, an rAAV transfection was performed.

The transient transfection of synthetic miRNA mimics aims to achieve miRNA overexpression, followed by a 48h incubation period, to allow for the respective metabolic changes.

### ii) Mi/siRNA expression plasmid transfection of HEK293 suspension cells in the 96-well format

HEK293 suspension cells in BalanCD HEK293 medium supplemented with 4 mM L-Glutamine from a 3-day culture were seeded in cell-repellent 96-well plates (Greiner Bio-One GmbH) with a seeding density of 2.00E+04 vc in 90 µL per well (NucleoCounter^{®} NC-200^{™}, Chemometec). A DNA concentration of 0.10 µg/well (2.50 pg/vc) was applied with a PEI^{®}pro (Polyplus):DNA ratio of 1:1. DNA and PEI^{®}pro were diluted accordingly in fresh, supplemented BalanCD to reach a final volume of 10 µL per well each. Transfection mixes were prepared by adding PEI^{®}pro to the respective DNA and incubating for 20 min at room temperature. 20 µL transfection mix were added to each well preparing biological triplicates for each transfection approach. The 96-well plates were incubated at 37°C, 5% CO₂, 600 rpm on an orbital shaker with a 3 mm orbit (VWR) for 48h. After this incubation time, an rAAV transfection was performed.

As with the transient transfection of synthetic miRNA mimics, transfection with miRNA expressing plasmids aimed to achieve miRNA overexpression, followed by a 48h incubation period, to allow for the respective metabolic changes.

### iii) Mi/siRNA expression plasmid transfection of HEK293 suspension cells in an Ambr^{®}15 cell culture bioreactor

HEK293 suspension cells in BalanCD HEK293 medium supplemented with 4 mM L-Glutamine from a 1-day culture were seeded with a seeding density of 2.50E+06 vc/mL (NucleoCounter^{®} NC-202^{™}, Chemometec) in a total volume of 11 mL.

The mi/siRNA expression plasmids were diluted in fresh, supplemented BalanCD to a DNA concentration of 0.70 pg/vc in a final volume of 500 µL per bioreactor. PEIpro^{®} was diluted in fresh, supplemented BalanCD medium to a PEIpro^{®}:DNA ratio of 1:1 in a final volume of 500 µL per bioreactor. The PEIpro^{®}-mix was added to the DNA-mix and incubated for 20 min at room temperature. 1 mL transfection mix was added per bioreactor. The bioreactors were incubated for 41h before performing the subsequent rAAV transfection. As with the above-mentioned steps, transfection with miRNA expressing plasmids aimed to achieve miRNA overexpression to allow for the respective metabolic changes.

### iv) rAA V transfection of HEK293 suspension cells in the 96-well format

Transient rAAV transfections of HEK293 suspension cells in the 96-well format were performed 48h post RNA / mi/siRNA expression plasmid transfection. The recombinant AAV (AAV S3 serotype) transfection was performed utilising the 2-plasmid split system, described in detail in WO2020/208379 A1, EPEP3722434 B1 and WO 2022/079429 A1 (incorporated herein by reference). which comprises two plasmids encoding adenoviral helper functions and AAV rep on one plasmid and AAV cap and the respective transgene cassette on a second plasmid was used. The molar ratio between the respective plasmids was 1:3 (Ad/rep:cap/transgene). DNA concentrations of 0.20 µg/well (MP01 to MP12) or 0.40 µg/well (MP13 to MP33) were applied during the miRNA screen. A DNA concentration of 0.10 µg/well was applied during the transient validation. The PEI^{®}pro:DNA ratio was 1:1.

DNA and PEI^{®}pro were diluted accordingly in fresh, supplemented BalanCD. Transfection mixes were prepared by adding PEI^{®}pro to the respective DNA and incubating for 20 min at room temperature. During the miRNA screen 20 (MP01 to MP12) or 40 µL (MP13 to MP33) transfection mix were added to each well. During the transient validation 20 µL transfection mix were added to each well.

After 48h incubation to allow for rAAV production, cells were harvested and a freeze-thaw lysis was performed by repeated freezing and thawing at -80°C and 37°C, respectively (repeated three times). The 96-well plates were centrifuged at 3700x g, room temperature for 15 min before transferring the supernatant to a new 96-well plate. The clarified freeze-thaw lysate was directly used for the subsequent transduction of Huh7 cells.

At this point rAAV production was analyzed e.g., via rAAV vector genome titration. However, additional steps are required to clear residual plasmid and genomic DNA, rendering rAAV vector genome titration directly from clarified rAAV lysate highly susceptible to noise and not feasible for high throughput. To this end, the production of functional rAAV particles was analyzed after a transduction applying clarified rAAV lysate without prior purification or titration. Additionally, this facilitates the identification of miRNAs improving not only rAAV production but potentially also vector efficiency.

### v) rAA V transfection of HEK293 suspension cells in the Ambr^{®}15

Transient rAAV transfections of HEK293 suspension cells in the Ambr@15 were performed 41h post mi/siRNA expression plasmid transfection. Cell suspension of all bioreactors was sampled for a cell count and viability determination using the NucleoCounter^{®} NC-202^{™} (Chemometec). Based on the determined cell density a removal of cell suspension via rapid vessel drain and subsequent refill with pre-warmed, supplemented BalanCD HEK293 medium was performed to obtain a cell density of 1.25E+06 vc/mL (first transfection) or 2.00E+06 vc/mL (second and third transfection) in a total volume of 12 mL.

As above the 2-plasmid split system was used for thetransient rAAV transfections. The molar ratio between the respective plasmids was 1:3 (Ad/rep:cap/transgene). A DNA concentration of 0.50 pg/vc and a PEIpro^{®}:DNA ratio of 1:1 were applied. DNA and PEIpro^{®} were diluted in fresh, supplemented BalanCD HEK293 medium in a final volume of 650 µL per bioreactor each. The PEIpro^{®}-mix was added to the DNA-mix and incubated for 20 min at room temperature. 1.3 mL transfection mix was added per bioreactor. The bioreactors were incubated for 48h before the harvest and freeze-thaw lysis, as described above in (iv).

### vi) Transduction of Huh7 cells in the 96-well format

Based on the liver-specificity of AAVS3 the transduction was performed with Huh7 cells. To this end Huh7 cells from a 4-day culture were seeded in Nunclon Delta Surface 96-well plates (ThermoFisher) at a seeding density of 2.5E4 vc in 200 µL cultivation medium per well. The cultivation medium consisted of DMEM (low glucose), 10% FBS and 1x GlutaMax. The cells were incubated for 5h at 37°C, 5% CO₂ before being treated with 10 µg/mL Mitomycin C in 100 µL cultivation medium per well. The Mitomycin C treatment was performed for 1h at 37°C and 5% CO₂. Afterwards Huh7 cells were washed with 100 µL transduction medium consisting of DMEM (low glucose) and 1x GlutaMax before adding 100 µL transduction samples and controls comprised of three parts clarified rAAV lysate and seven parts transduction medium into the respective wells. The 96-well plates were incubated at 37°C, 5% CO₂ overnight.

Approximately 17 hours post-transduction, Huh7 cells were washed with 100 µL transduction medium per well and after supernatant removal 200 µL medium consisting of DMEM (low glucose), 5% FBS and 1x GlutaMax were added per well. After a 48h incubation period at 37°C, 5% CO2, the supernatant was harvested by centrifuging transduction plates at 150 x g, room temperature for 5 min. 150 µL supernatant per well was transferred to a new Nunclon Delta Surface 96-well plate (ThermoFisher). The supernatant was used directly for analysis via SEAP assay or stored at -80°C.

### vii) SEAP quantification

Secreted alkaline phosphatase (SEAP) was chosen as a read-out marker, since it is a widely used reporter with several advantages, ranging from the simplicity of sample collection directly from the supernatant of cells to its negligible impact on transfected cells, a high dynamic range, and the quenchable background of endogenous alkaline phosphatase due to its heat-stability.

Quantification of secreted alkaline phosphatase protein (SEAP) was performed using the Great EscAPe^{™} SEAP Chemiluminescence Kit 2.0 (TaKaRa Bio) applying recombinant SEAP (rSEAP, InVivoGen) as standard and trending control. rSEAP was diluted in spent Huh7 cell culture medium consisting of DMEM low glucose, 5 % FBS and 1 % GlutaMAX after a 48h growth period of Huh7 cells to obtain a standard curve ranging from 1.33E+06 to 1.83E+03 pg/mL and a trending control with 5.00E+05, 5.00E+04 and 5.00E+033 pg/mL. A 1:4 dilution in 1x dilution buffer was prepared for the standard curve, trending control, and all samples. To inactivate endogenous SEAP, an incubation at 65°C was performed for 30 min before equilibrating samples and controls to RT. During the miRNA screen 100 (MP01 to MP03) or 50 µL (MP04 to MP33) Substrate Solution were added to 100 or 50 µL of samples and controls, respectively. During the transient validation 100 µL Substrate Solution were added to 100 µL samples and controls. A 30 min incubation was performed at room temperature in the dark. Luminescence measurement was performed with the Infinite^{®} 200 Pro (Tecan; miRNA screen) or the SpectraMax M3 multi-mode microplate reader (Molecular Devices, transient validation) according to the manual.

### viii) rAAV vector genome quantification

The rAAV vector genome assay is based on a quantitative polymerase chain reaction (qPCR) specific for the promoter sequence of the rAAV expression cassette. In principle, the qPCR primers can be designed to bind any part of the recombinant AAV genome which is not common to wild type AAV genomes, but is it recommended against using primer template sequences very close to the ITRs as doing so can lead to an exaggerated vector genome titre measurement. Cell lysate test samples were subjected to a nuclease treatment procedure in order to remove non-packed vector genomes prior to performing the qPCR. To that aim the samples were pre-diluted 1:100 in nuclease-free water containing 0.125% Pluronic F-68. 25 µl of the pre-dilution were used for the digest with 2 units of Turbo DNase (ThermoFisher Scientific, Waltham, USA) and 1x Turbo DNase reaction buffer, resulting in a total reaction volume of 29 µl. Incubation was performed for 30 min at 37°C. Afterwards, 1 volume of 0.1 M EDTA was added and the samples were incubated for 10 min at 75°C. To control for the quality of the Turbo DNase digest, a trending control containing unpurified cell lysate with a known AAV vector genome titre and spike-in controls using plasmid DNA carrying the promoter sequence were measured in parallel.

Alternatively, a Denarase treatment was performed by adding Denarase^{®} (100 U/mL; C-Lecta) to the complete volume of clarified rAAV lysate to a final concentration of 0.02 U/µL and incubating over night at RT.

Per sample, 10 µl 2x iTaq Universal SYBR Green Supermix (Biorad) were mixed with 0.80 µl of qPCR primer working stock solution (containing 10 mM of each primer) and filled up to a volume of 15 µl with nuclease-free water. 5 µl of pre- treated cell lysate or purified virus test sample were added to the mix (total reaction volume 20 µl, final primer concentration in the reaction 400 nM each) and qPCR was performed in a CFX 96 Touch Real Time PCR cycler (Bio-Rad Laboratories Inc., Hercules, USA) with following program steps: 95°C 5 min; 39 cycles (95°C 5 s, 60°C 30 s, plate read); 95°C 10 sec; 60-95°C (+0.5°C/step), 10 sec; plate read. To control for the quality of the qPCR, a trending control with known rAAV vector genome titre was measured in parallel. To check for contaminations, a no template control (NTC, 5 µl H2O) was also included. Standard row, test samples and controls were measured in triplicates for each dilution. Purified virus test samples and trending control were generally measured in 3 different dilutions in EB buffer (10 mM Tris-Cl, pH 8.5). TurboDNase treated rAAV lysate test samples were applied in the qPCR in a 1:10 dilution in EB buffer. Denarase^{®} treated rAAV lysate test samples were applied in the qPCR in 1:1000, 1:10000 and 1:100000 dilutions. Data were analysed using the CFX ManagerTM Software 3.1 (Bio-Rad Laboratories Inc.). Melting curve analysis confirmed the presence of only one amplicon. Amplification results in nascent double stranded DNA amplicons detected with the fluorescent intercalator SYBR Green to monitor the PCR reaction in real time. Known quantities of the promoter genetic material, in the form of a linearised plasmid, were serially diluted to create a standard curve and sample vector genome titre was interpolated from the standard curve.

### ix) Quantification of rAAV particles (capsids)

rAAV capsids were quantified using the automated immunoassay platform Gyrolab xPlore^{™} (Gyros Protein Technologies AB, Uppsala, Sweden) in conjunction with the Gyrolab^{®} AAVX Titer Kit. The sandwich immunometric technique used to capture and detect AAVS3 capsids is based on the Thermo Scientific^{™} CaptureSelect^{™} Biotin Anti-AAVX Conjugate and Thermo Scientific CaptureSelect Alexa Fluor^{™} 647 Anti-AAVX Conjugate from Thermo Fisher Scientific (Waltham, Massachusetts, USA). A serial dilution of AAVS3 control (in-house) was applied to interpolate AAVS3 concentrations in the test samples. An AAVS3 positive control / trending control (in-house) was applied in three dilutions covering the upper, medium, and lower assay range. A negative control was included to check for contamination of reagents. Denarase^{®} treated cell lysates, purified virus test samples and AAVS3 control serial dilution were diluted in Gyrolab^{®} AAVX Titer Sample Dilution Buffer. All samples and controls were tested in duplicate. Data were analyzed using the Gyrolab^{®} Evaluator Software Version 3.7.1.252.

### x) Quantification of plasmid-derived impurities

Prokaryotic DNA sequences, such as antibiotic resistance genes or parts of them originating from the bacterial backbone of the producer plasmids, can be packaged into the rAAV particles, constituting product-related impurities. Plasmid-derived impurity quantification is based on experiments using qPCR techniques specific for defined sequences of the kanamycin resistance gene (kanR), present on both helper and vector plasmid, and the AAV cap gene, which is present on the vector plasmid. Before performing the plasmid-derived impurity qPCRs, nuclease treatment of clarified, freeze-thawed cell lysates was performed. An AVB-spin (batch) procedure was then performed using POROS^{™} CaptureSelect^{™} AAVX Affinity Resin (ThermoFisher Scientific). Plasmid-derived impurity qPCRs were performed on the purified material. The plasmid-derived impurity qPCRs were performed according to the method for *"quantification of rAAV vector genomes"* outlined above with appropriate modifications to reflect the impurity templates, such as the primer pairs, standards and controls. Specifically, modifications were made to the impurity specific primer pairs, the linearized plasmid standards, the trending controls, sample dilution and the annealing temperatures (57°C) in the qPCR setup.

### xi) Quantification of host cell-derived impurities

DNA sequences, such as nucleic acid material from the genome of host cells in which rAAV are produced, can be packaged into rAAV particles, constituting product-related impurities. Host cell-derived impurity quantification was carried out using droplet digital polymerase chain reaction (ddPCR) specific for defined sequences of the 18S rRNA gene locus of the 45S ORF present in HEK293T cells. Clarified and freeze-thawed cell lysates were nuclease treated. An AAVX-spin (batch) procedure was then performed using POROS^{™} CaptureSelect^{™} AAVX Affinity Resin. Host cell-derived impurity ddPCR was performed on the purified material.

The rAAV vector samples were diluted in EB (10mM Tris-Cl, pH 8.5) in a range of 1:5 to 1:40. The reaction mixtures were prepared using ddPCR Supermix for Probes (1863026, Bio-Rad) with 0.9 µM primers specific for the 18S rRNA gene locus and 0.25 µM probe specifically binding to the 18S rRNA gene locus and 8 µL sample dilution in a final volume of 20 µL as described in the manufacturer's protocol. Samples were partitioned with droplet generator oil for probes (Bio-Rad) using a QX-200 droplet generator (Bio-Rad) according to manufacturer's instructions.

PCR amplification of the droplets was performed using a thermal cycler with the following parameters: 95°C for 10 min, followed by 50 cycles of 94°C for 30 s, 59°C for 65 s followed by a final 98°C heat treatment for 10 min. Droplet read-out of the PCR plate wells was carried out on the QX200 droplet reader (Bio-Rad) and data were analyzed with QuantaSoft software (Bio-Rad). To control for the quality of the ddPCR, a trending control was measured in parallel. To check for contaminations, a no template control (NTC, EB) was also included. Test samples and controls were measured in duplicates for each dilution.

### xii) Purification of rAA V

Harvest of cell lysate was treated with Denarase (100 U/mL) and filtrated using a 0.45 mm bottle top filter. AAVX (adeno-associated virus binding epitope) affinity chromatography was performed in a batch protocol using POROS^{™} CaptureSelect^{™} AAVX Affinity Resin (ThermoFisher Scientific)

### B: RESULTS

### i) Effect of the transfected miRNAs on rAAV production

The functionality of transfected miRNA mimics was shown by the ability to silence a specific target gene. A suitable model miRNA gene, that has been applied in human adipocytes and CHO cells, is hsa-miR-1-3p which has been suggested to bind to the 3'UTR of the twinfilin1 (twf1) transcript and induce its mRNA degradation. With the established RNA transfection parameters for the HEK293 suspension cells (0.2 µL/well Lipofectamine^{®} 3000, 100 nM RNA/well) hsa-miR-1-3p resulted in a downregulation of twf1 mRNA to approx. 0.6-fold in two independent experiments, thereby indicating a robust biological function of transfected miRNAs (Figure 1A).

The rAAV transfection control without preceding RNA transfection, which monitors production of functional rAAV particles without preceding RNA transfection, showed the highest rAAV vector genome (Figure 1B) and capsid titers (Figure 1D) with significantly decreasing rAAV titers upon performing an RNA and rAAV double transfection as indicated by the NT-siRNA. This was attributed to a cytotoxic effect of Lipofectamine^{®} 3000 on the HEK293 suspension cells as well as an increasing HEK293 aggregation after the initial RNA transfection reducing rAAV transfection efficiency. The rAAV-specific negative trending control, cap_1990 siRNA, resulted in a further reduction of rAAV vector genome and capsid titers, which was confirmed upon normalization against the respective NT-siRNA (Figure 1C, 1E). This was due to the cap_1990 siRNA-mediated downregulation of AAV capsid mRNA.

Functional rAAV particles showed the same trends as described for rAAV titers (Figure 1F, 1G). This final SEAP read-out comprised two additional trending controls - the SEAP assay trending control monitoring the SEAP assay and the rAAV transduction control monitoring the transduction of Huh7 cells. The rAAV transduction control exhibited the highest SEAP concentration due to the larger production format (shake flask) and different rAAV transfection parameters for the respective transfection formats.

In summary, the miRNA screening process of the present disclosure shows a successful RNA transfection in combination with a successful rAAV production.

### ii) Results of the rAAV-specific miRNA screening

The miRNA screening process using the Human miRIDIAN miRNA Mimic Library (Horizon Discovery) was performed as described in (A) above, with biological triplicates applying data normalization against the respective on-plate NT-siRNA to allow for inter-plate comparisons. Significant changes in SEAP expression were determined via a one-way analysis of variances (ANOVA) combined with a Dunnett's multiple comparison test (against NT-siRNA; p<0.05).

6.55% (160) of the transfected miRNAs increased the production of functional rAAV particles in the rAAV-specific miRNA screen by at least 2-fold with selected miRNAs showing improvements of up to 24-fold (Figure 2A, 2B). Most of these positive miRNAs (96) showed between 2- to 4-fold increases in rAAV production.

In order to confirm the reproducibility of miRNA-mediated effects data from MP01 to MP06 were used, which corresponded well to the results of the complete rAAV-specific miRNA screen with 6.67% (32) of transfected miRNAs increasing the production of functional rAAV particles by at least 2-fold with selected candidates showing up to 10-fold improvements (Figure 2C, 2D). 19 of these 32 miRNAs were applied in the first round of transient validation. The remaining 13 candidates were excluded from a more detailed analysis due to the NT-siRNA on the respective assay plate being out of range in the corresponding SEAP assay thereby increasing the risk of identifying false-positive candidates. Additionally, two miRNAs with a negative impact on rAAV production as well as two neutral miRNAs from master plates MP01 and MP06 were included in the transient validation guaranteeing the reproducibility of the complete range of miRNA-mediated effects.

This comprised confirmation experiments in the 96-well format together with upscaling to the automated Ambr@15 microbioreactor system, which is routinely applied for the development and optimization of starting materials and bioprocess and serves as downscaling model for large-scale bioreactors relevant for the commercial production of rAAV. For that purpose, miRNA expression plasmids enabling a transient and stable expression of miRNAs in mammalian cells and widely used as alternative to miRNA mimics were applied. Moreover, the application of miRNA expression plasmids also served as a preparation for the generation of cell lines stably overexpressing miRNAs. To this end, the use of commercially available pcDNA^{™}6.2-GW/EmGFP-miR miRNA expression plasmids from the BLOCK-iT^{™} Pol II miR RNAi Expression Vector Kit (ThermoFisher) was established for DNA and rAAV double transfections of the HEK293 suspension cells in the Ambr@15. Here, miRNA expression driven by a hCMV promoter is integrated into the transcription unit of Emerald Green Fluorescent Protein (EmGFP). To increase mature miRNA expression together with the ability to silence the respective target gene four copies of the respective miRNA encoding sequences were serially cloned into the expression plasmids (miRNA chaining). pcDNA^{™}6.2-GW/EmGFP-miR encoding four copies of the miR negative control from the BLOCK-iT^{™} Pol II miR RNAi Expression Vector Kit was applied as non-targeting control to facilitate data normalization.

For a continuous transient validation of miRNAs enabling the differentiation between miRNA transfection systems and transfection formats a miRNA expression plasmid and rAAV double transfection was also implemented in the 96-well format.

Taken together, the availability of these three transient validation systems produced the following transient validation strategy: First, miRNA screen data was confirmed via transient miRNA mimic transfections in the 96-well format (Figure 3A). Second, the same miRNAs were analysed in the 96-well format in the form of miRNA expression plasmids (Figure 3B). Lastly, the scalability of miRNA-mediated effects on rAAV production was assessed via miRNA expression plasmid transfections in the Ambr@15 (Figure 3C). Overall, Figure 3 depicts the results of the transient validation across the three systems for four selected miRNAs from MP01 to MP06 - hsa-miR-125a-5p (SEQ ID NO:1), hsa-miR135b-5p (SEQ ID NO:2), hsa-miR-519c-5p (SEQ ID NO: 3) and hsa-miR-563 SEQ ID NO: 4.

**Table 1. miRNA sequence table**

| **Molecule name** | **Molecule type** | **SEQ ID NO** | **SEQUENCE** |
|---|---|---|---|
| hsa-miR-125a-5p | miRNA | 1 | UCCCUGAGACCCUUUAACCUGUGA |
| hsa-miR-135b-5p | miRNA | 2 | UAUGGCUUUUCAUUCCUAUGUGA |
| hsa-miR-519c-5p | miRNA | 3 | CUCUAGAGGGAAGCGCUUUCUG |
| hsa-miR-563 | miRNA | 4 | AGGUUGACAUACGUUUCCC |
| cap_1990 siRNA | siRNA | 5 | CCGGCCAAGUUUGCUUCAUUUAUCA |

**Table 2. Plasmid table**

| **Molecule type** | **SEQ ID NO** |
|---|---|
| Plasmid encoding hsa-miR-125a-5p | 6 |
| Plasmid encoding hsa-miR135b-5p | 7 |
| Plasmid encoding hsa-miR-519c-5p | 8 |
| Plasmid encoding hsa-miR-563 | 9 |
| Plasmid encoding cap_1990 siRNA | 10 |

All four miRNAs show a positive impact on the production of functional rAAV particles (namely particles able to transduce a selected target cell line) in the miRNA screening, which could be confirmed via at least one independent miRNA mimic transfection (Figure 3A). The positive impact on rAAV production could be confirmed for miR-125a-5p, miR-519c-5p and miR-563 via three independent transient transfections of miRNA expression plasmids in the 96-well format (Figure 3B). No significant impact on rAAV production was observed for miRNA-135b-5p in the form of a miRNA expression plasmid, which was attributed to the differences between the respective miRNA transfection systems. Most importantly, all four miRNAs exhibited a scalable increase in functional rAAV particles between 1.2- to 1.6-fold in the Ambr@15 (Figure 3C). Overall, the positive miRNA-mediated effect on rAAV production was lower in the Ambr@15 compared to the 96-well format. This observation can be attributed to differences between transfection systems with the Ambr@15 featuring an inherently higher rAAV production due to the numerous advantages of tightly controlled bioreactors in contrast to uncontrolled small-scale formats such as 96-well plates.

Additionally, Figure 4 shows the effect of the four selected miRNAs on produced rAAV capsid titres (Fig.4A), rAAV vector genome titers (Fig. 4B) and functional rAAV particles (Fig. 4C) during rAVV production in the Ambr@15 bioreactor system. Figure 4D shows a direct comparison between the three read-out methods with very comparable results. This indicated that the respective miRNAs directly affected rAAV production instead of rAAV transduction efficiency.

Plasmid-derived cap (Figure 5A) and kanR (Figure 5B) as well as host cell-derived impurities indicated via 18S rRNA (Figure 5C) were analyzed to assess the potential impact of these four selected miRNAs on rAAV quality. The four miRNAs showed no detectable difference in any of these rAAV packaging impurities in comparison to the miR negative control plasmid. This highlighted the fact increases in rAAV quantity could be obtained while maintaining a consistently high rAAV quality.

## Claims

1. A method for producing a preparation comprising recombinant adeno-associated virus (rAAV) particles, wherein said method comprises a step of introducing a microRNA molecule or a microRNA expressing vector to a host cell under suitable conditions, so that said microRNA molecule is overexpressed, and a step of culturing said host cell under suitable conditions to produce the preparation; and wherein said preparation comprises rAAV particles having improved and/or increased viral genome titre, capsid titre, and/or potency compared to a preparation produced by a corresponding method without introducing said same microRNA molecule or said same microRNA expressing vector.

2. A method according to claim 1, wherein said method comprises the steps of
- transfecting a host cell line, preferably a mammalian cell line, more preferably a human cell line, with a synthetic microRNA molecule or a microRNA expressing vector
- transfecting said host cell line with a recombinant AAV production plasmid system, preferably a three plasmid rAAV production system or a two plasmid rAAV production system
- culturing said cell line under conditions suitable for recombinant AAV production
- obtaining recombinant AAV particles from said transfected cells,
wherein said preparation comprises rAAV particles having increased viral genome titre, capsid titre and/or potency compared to a preparation produced by a corresponding method without transfection with said same synthetic microRNA molecule or said same microRNA expressing vector.

3. A method according to any one of claims 1 to 2, wherein said increase is at least 1.05 fold, at least 1.1 fold, at least 2 fold, at least 3 fold, at least 4 fold, at least 5 fold or at least 6 fold compared to a corresponding method without introducing said same microRNA molecule or said same microRNA expressing vector or without transfection with said same synthetic microRNA molecule or said same microRNA expressing vector.

4. A method for improving and/or increasing the viral genome titre, capsid titre and/or potency of recombinant adeno-associated virus (rAAV) particles produced during recombinant AAV production, wherein said method comprises a step of introducing a microRNA molecule or a microRNA expressing vector to a host cell under suitable conditions so that said microRNA molecule is overexpressed; and a step of culturing said host cell line under suitable conditions to produce said rAAV, and wherein said increase is at least 1.05 fold, at least 1.1 fold, at least 2 fold, at least 3 fold, at least 4 fold, at least 5 fold or at least 6 fold compared to a corresponding method without introducing said same microRNA molecule or said same microRNA expressing vector

5. A method for improving and/or increasing the viral genome titre, capsid titre and/or potency of recombinant adeno-associated virus (rAAV) particles produced during recombinant AAV production according to claim 4 comprising the steps of
- transfecting a host cell line with a synthetic microRNA molecule or a microRNA expressing vector
- transfecting said cell line with a recombinant AAV production plasmid system, preferably a two - plasmid system
- culturing said host cell line under conditions suitable for recombinant AAV production
- obtaining recombinant AAV particles from said transfected cells,
wherein said increase is at least 1.05 fold, at least 1.1 fold, at least 2 fold, at least 3 fold, at least 4 fold, at least 5 fold or at least 6 fold compared to a corresponding method without transfection with said same synthetic microRNA molecule or said same microRNA expressing vector.

6. The method of claim 5, wherein said microRNA expressing vector comprises at least 2, at least 3, or at least 4 copies of a sequence encoding said microRNA.

7. The method of any one of claims 4 to 6, wherein said microRNA comprises or consists of the sequence of any one of SEQ ID NOs: 1 to 4.

8. The method of any one of claims 1 to 7, wherein said host cell line is a HEK293 cell line, preferably a suspension HEK293 cell line.

9. The method of any one of claims 2 to 8, wherein said two- plasmid system comprises a helper plasmid comprising at least one adeno-associated virus (AAV) rep gene encoding at least one functional AAV Rep protein and at least one helper virus gene, and which does not comprise a cap gene encoding a functional set of Cap proteins, and a vector plasmid comprising an AAV cap gene encoding at least one functional AAV Cap protein.

10. A method of identifying a microRNA which improves and/or increases the viral genome titre, capsid titre and/or potency of recombinant adeno-associated virus (rAAV) produced during recombinant AAV production comprising the steps of
- transfecting a first host cell line with a synthetic microRNA molecule or a microRNA expressing vector
- transfecting said host cell line with a recombinant AAV plasmid system, preferably a two -plasmid system
- culturing said cell line under conditions suitable for recombinant AAV production
- obtaining recombinant AAV particles from said transfected cells,
- analysing the functionality of said AAV particles, preferably by transducing a second cell line with a lysate obtained after lysis of said AAV particles, wherein said second cell line is selected based on the AAV serotype specificity, and
wherein said increase of said is at least 1.05 fold, at least 1 fold, at least 2 fold, at least 3 fold, at least 4 fold, at least 5 fold or at least 6 fold compared to a corresponding method without transfection with said same synthetic microRNA molecule or said same microRNA expressing vector.

11. The method of claim 10, wherein said microRNA expressing plasmid comprises at least 2, at least 3, or at least 4 copies of a sequence encoding said microRNA.

12. The method of any one of claims 10 to 11, wherein said microRNA comprises or consists of the sequence of any one of SEQ ID NOs: 1 to 4.

13. The method of any one of claims 10 to 12, wherein said first cell line is a HEK293 cell line, preferably a suspension HEK293 cell line.

14. The method of any one of claims 10 to 13, wherein said two- plasmid system comprises a helper plasmid comprising at least one adeno-associated virus (AAV) rep gene encoding at least one functional AAV Rep protein and at least one helper virus gene, and which does not comprise a cap gene encoding a functional set of Cap proteins, and a vector plasmid comprising an AAV cap gene encoding at least one functional AAV Cap protein.

15. A cell line for use in a method for producing a preparation comprising recombinant adeno-associated virus (rAAV) particles according to any one of claims 1 to 3, or method for improving and/or increasing the viral genome titre, capsid titre and/or potency of recombinant adeno-associated virus (rAAV) produced during recombinant AAV production according to any one of claims 4 to 9 or a method of identifying a microRNA which improves and/or increases the viral genome titre, capsid titre and/or potency of recombinant adeno-associated virus (rAAV) produced during recombinant AAV production according to any one of claims 10 to 14, wherein said cell line expresses a microRNA comprising or consisting of the sequence of any one of SEQ ID NOs: 1 to 4.

16. Use of a microRNA molecule in a method for producing a preparation comprising recombinant adeno-associated virus (rAAV) particles according to any one of claims 1 to 3 or method for improving and/or increasing the viral genome titre, capsid titre and/or potency of recombinant adeno-associated virus (rAAV) produced during recombinant AAV production according to anyone of claims 4 to 9 or a method of identifying a microRNA which improves and/or increases the viral genome titre, capsid titre and/or potency of recombinant adeno-associated virus (rAAV) produced during recombinant AAV production according to any one of claims 10 to 14, wherein said microRNA comprises or consists of the sequence of any one of SEQ ID NOs: 1 to 4.
